(19) Europäisches Patentamt
European Patent Office
Office européen des brevets

(11) EP 1 060 187 B1

(12) **EUROPÄISCHE PATENTSCHRIFT**

(45) Veröffentlichungstag und Bekanntmachung des Hinweises auf die Patenterteilung:
**16.04.2003 Patentblatt 2003/16**

(21) Anmeldenummer: **99915475.0**

(22) Anmeldetag: **10.02.1999**

(51) Int Cl.[7]: **C07J 41/00**, A61K 31/565, C07J 43/00

(86) Internationale Anmeldenummer:
**PCT/DE99/00408**

(87) Internationale Veröffentlichungsnummer:
**WO 99/045023 (10.09.1999 Gazette 1999/36)**

(54) **S-SUBSTITUIERTE 11BETA-BENZALDOXIM-ESTRA-4,9-DIEN-KOHLENSÄURETHIOLESTER, VERFAHREN ZU DEREN HERSTELLUNG UND DIESE VERBINDUNGEN ENTHALTENE PHARMAZEUTISCHE ZUBEREITUNGEN**

S-SUBSTITUTED 11BETA-BENZALDOXIME-ESTRA-4,9-DIENE-CARBONIC ACID THIOLESTERS, METHOD FOR THE PRODUCTION THEREOF AND PHARMACEUTICAL PREPARATIONS CONTAINING THESE COMPOUNDS

THIOLESTERS D'ACIDE 11BETA-BENZALDOXIM-ESTRA-4,9-DIENE-CARBONIQUE A SUBSTITUTION S, LEUR PROCEDE DE PRODUCTION ET PREPARATIONS PHARMACEUTIQUES CONTENANT CES COMPOSES

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **03.03.1998 DE 19809845**

(43) Veröffentlichungstag der Anmeldung:
**20.12.2000 Patentblatt 2000/51**

(73) Patentinhaber: **Schering Aktiengesellschaft
13353 Berlin (DE)**

(72) Erfinder:
- **SCHUBERT, Gerd
D-07743 Jena (DE)**
- **RING, Sven
D-07749 Jena (DE)**
- **KAUFMANN, Günther
D-07743 Jena (DE)**
- **ELGER, Walter
D-14195 Berlin (DE)**
- **SCHNEIDER, Birgit
D-07745 Jena (DE)**

(74) Vertreter: **Ziebig, Marlene, Dr. Dipl.-Chem.
Patentanwälte
Gulde Hengelhaupt Ziebig & Schneider
Schützenstrasse 15-17
10117 Berlin (DE)**

(56) Entgegenhaltungen:
**EP-A- 0 648 778**
**EP-A- 0 648 779**
**WO-A-96/12494**
**WO-A-96/19997**
**WO-A-96/28145**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue S-substituierte 11β-Benzaldoxim-estra-4,9-dien-kohlensäurethiolester der allgemeinen Formel I,

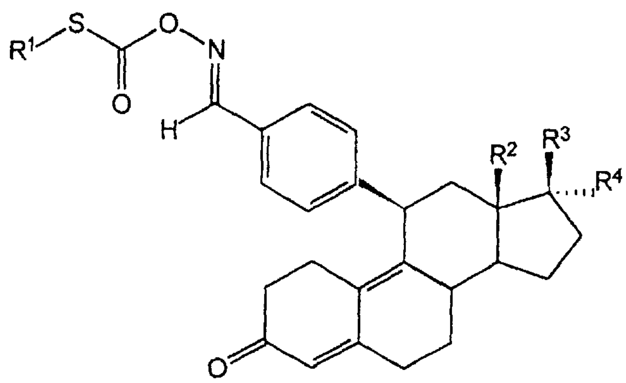

(I)

und deren pharmazeutisch annehmbare Salze, ein Verfahren zu deren Herstellung und diese Verbindungen enthaltende pharmazeutische Zubereitungen.

Aus der EP-A-0 648 778 und EP-A-0 648 779 sind Ester, Kohlensäureester und Urethane von 11β-Benzaldoxim-estra-4,9-dienen bekannt. Die darin beschriebenen Verbindungen haben antigestagene Wirkung.

Antigestagene sind Steroide, die wie Progesteron und andere gestagene Substanzen hohe Affinität zum Progesteronrezeptor aufweisen. Anders als diese führen sie aber nicht zu den typischen durch den Progesteronrezeptor vermittelten physiologischen Effekten. Vielmehr wird Progesteron aus seiner Bindung an den Rezeptor verdrängt und seine Wirkung gehemmt. Es ist aus der wissenschaftlichen Literatur bekannt, daß hierbei neben der Verdrängung von Progesteron von seiner Bindungsstelle Störungen der genregulatorischen Rezeptorfunktion eine entscheidende Rolle spielen (Klein-Hitpass, L., Cato, A.C.B., Henderson, D., Ryffel, U.: Nucleic Acid Res. **19** (1991), 1227-1234 ; Horwitz, K. B.: Endocrine Rev. **13** (1992) 146); McDonnell D.P.: Trends Endocrinol. Metab. **6** (1995) 133-138).

Bezüglich des letztgenannten Aspektes unterscheiden sich bekannte Antagonisten, zum Beispiel ZK 98299 = Onapriston (DE-OS-35 04 421) und RU 486 = Mifepriston (EP-A-0 057 115) auf molekularer Ebene (Typ I - / Typ II - Antagonisten) und zwar dadurch, daß bei Typ I - Antagonisten (z.B. Onapriston) der Hormonrezeptorkomplex nicht mehr oder labil an die hormonresponsiblen Elemente der DNA bindet, während dies beim Typ II (z.B. RU 486) noch der Fall ist (Klein-Hitpass et al.). Antigestagene, die Rezeptorbindung an DNA noch zulassen, können progesteronähnliche Wirkungen haben, während dies bei Störung der DNA-Bindung des Rezeptors nicht möglich ist.

Eine Modulation genregulatorischer Aktivität einzelner Progesteronantagonisten kann auch durch Mechanismen erfolgen, die zunächst am Rezeptorprotein angreifen. Verschiedene Arbeiten haben nachgewiesen, daß Antagonist-Rezeptorkomplexe durch zyklisches AMP in ihrer genregulatorischen Aktivität stimuliert werden. Bei Vorliegen hoher Konzentrationen von c-AMP im Gewebe tritt eine Aktivierung der Antagonist-Rezeptorkomplexe in Erscheinung, bei niedrigen Konzentrationen bleibt der Rezeptor hinsichtlich genregulatorischer Aktivität gehemmt. Das Auftreten entsprechender Phänomene ist offenbar ebenfalls substanzspezifisch. Die Erzeugung hoher c-AMP Konzentrationen (in vitro) führt bei manchen Antigestagenen zu partialagonistischer Wirkung, bei anderen Substanzen werden entsprechende Effekte durch c-AMP dagegen nicht ausgelöst (Sartorius, CA.,Tung, L., Takimoto, GS., Horwitz, KB.: J Biol. Chem **268** (1993) 9262-9266; Sobek, L., Kaufmann, G., Schubert, G., and Oettel, M.,: 79$^{th}$ Annual Meeting of the Endocrine Society **1997**, 3-452, 549).

Unterschiede auf der molekularen Ebene schlagen sich auch im pharmakodynamischen Verhalten von Progesteronantagonisten nieder. Dieses läßt sich am unterschiedlichen pharmakodynamischen Verhalten von Substanzen demonstrieren, die in vivo und in vitro sehr gut charakterisiert sind, wie Onapriston und Mifepriston (RU 486) [Elger, W., W., Neef, G., Beier, S., Fähnrich, M., Grundel, M. et al. in Current Concepts in Fertility Regulation and Reproduction, ed. Puri, C.P. and Van Look, P.F.H. (1994) 303-328.

Progesteron spielt in der Steuerung der an Reproduktionsvorgängen beteiligten Organsysteme eine entscheidende Rolle. Dies gilt für die morphologischen Umbauvorgänge im Genitaltrakt und in der Brustdrüse, die Regulation von Hormonen des Hypophysenvorderlappens und der Genitalorgane oder die Hemmung und Aktivierung von Geburtsvorgängen. Diese Funktionen reagieren auf Progesteron unterschiedlich empfindlich. Vorgänge, die sich bei sehr niedrigen Progesteronspiegeln abspielen verdienen im Hinblick auf die Pharmakologie der Antigestagene eine besondere Betrachtung. "Reine" Progesteronantagonisten des Typ I können Effekte bewirken, die mit partialagonistischen Antagonisten bei keiner Dosis erreicht werden können. Dies dürfte generell dann der Fall sein, wenn die Schwelle für den jeweiligen Effekt niedrig, das heißt unterhalb der partialagonistischen Aktivität eines Progesteronantagonisten liegt. Umgekehrt besteht die Möglichkeit, daß unter dem Einfluß von Progesteronantagonisten des Typ II Effekte beobachtet werden, die nicht durch die Hemmung, sondern durch die Aktivierung des Progesteronrezeptors ausgelöst werden. Bei gleicher Dosierung dieses Antagonisten werden die Funktionen des Progesterons gehemmt, die bei hohen Gewebskonzentrationen ablaufen.

Ein Beispiel für den erstgenannten Fall ist die Prostaglandinsekretion des Uterus beim Meerschweinchen im Zyklus. Diese wird gegen Zyklusende durch niedrigste Progesteronspiegel im Blut stimuliert. Nur reine Progesteronantagonisten des Typ I sind in der Lage, die Prostaglandinsekretion des Uterus bei Meerschweinchen so weit zu hemmen, daß die Rückbildung des Gelbkörpers völlig gehemmt wird (Elger, W., Neef, G., Beier, S., Fähnrich, M., Grundel, M. et al. in Current Concepts in Fertility Regulation and Reproduction, ed. Puri, C.P. and Van Look, P.F.H. **1994** 303-328). Partialagonistische Substanzen hemmen diesen Vorgang wenig oder garnicht.

Der Progesteronantagonist RU 486 hat beim Menschen verschiedene Effekte auf Funktionen der Fortpflanzung, die für den Einsatz in der Therapie relevant sind. Diese Substanz hemmt die Wirkung von Progesteron so stark, daß es bei ihrer Anwendung in der Gravidität zur Auslösung eines Abortes kommt. Diese abort- oder wehenauslösende Eigenschaft wird durch die simultane oder sequentielle Behandlung mit einem Prostaglandin erheblich verstärkt (Van Look, P.F.A.; Bygdeman,M.: Oxf. Rev. Reprod. Biol. **11** (1989),1-60 ; Elger, W., Neef, G., Beier, S., Fähnrich, M., Grundel, M. et al. in Current Concepts in Fertility Regulation and Reproduction, ed. Puri, C.P. and Van Look, P.F.H. **1994** 303-328). Entsprechende Effekte lassen sich auf der Basis der schwangerschafts-regulierenden Funktion des Progesterons in der Gravidität hinreichend erklären.

Daneben haben RU 486 und andere Antigestagene Effekte, bei denen der Mechanismus des Entzuges von Progesteron nicht so eindeutig gesichert ist. Dies betrifft in erster Linie Effekte im Zyklus in Phasen, in denen die Progesteronspiegel im Blut sehr niedrig sind. Hier sind zwei Phänomene besonders zu erwähnen, die Hemmung der Ovulation (Croxatto, H.B., Salvatierra; A.M.; Croxatto, H.D.; Fuentealba, A.: Hum. Reprod. **8** (1993), 201-207) und die Hemmung der oestrogeninduzierten Proliferation der Genitalepithelien, insbesondere die des Endometriums (Wolf, J. P., Hsiu, J.G., Anderson, T.L., Ulmann, A., Baulieeu, E.E. and Hodgen,G.D.: Fertility & Sterility **52** (1989) 1055-1060). Entsprechende Effekte sind von zentraler Bedeutung für den Einsatz der Antigestagene insbesondere für antiovulatorische Strategien in der Fertilitätskontrolle, die reversible Auslösung einer Amenorrhoe, zum Beispiel in der Therapie der Endometriose und zur Unterdrückung unerwünschter Oestrogeneffekte im Endometrium im Rahmen einer Substitutionstherapie mit Oestrogenen im Klimakterium. Für den therapeutischen Einsatz von Typ II-Antagonisten wie RU 486 ist die Kopplung der abortiven und wehenauslösenden Wirkung mit den Progesteron-agonistischen und insbesondere den antiovulatorischen und proliferationshemmenden Eigenschaften, von Nachteil.

Aufgabe der vorliegenden Erfindung ist es daher, Verbindungen zur Verfügung zu stellen, welche die oben geschilderten Nachteile überwinden.

Die Aufgabe wird dadurch gelöst, daß Verbindungen der allgemeinen Formel I

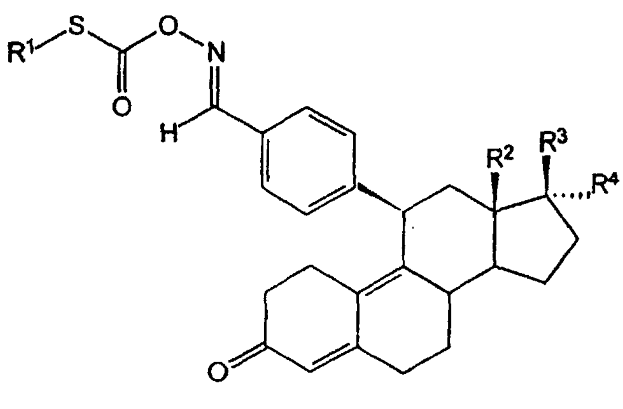

(I)

gemäß Anspruch 1 und ihre pharmazeutisch annehmbaren Salze sowie ein Verfahren zu ihrer Herstellung zur Verfügung gestellt werden. Ferner werden pharmazeutische Zusammensetzungen zur Verfügung gestellt, welche eine Verbindung der allgemeinen Formel I oder deren pharmazeutisch annehmbares Salz enthalten.

Gegenstand der vorliegenden Erfindung sind somit S-substituierte 11β-Benzaldoxim-estra-4,9-dienkohlensäurethiolester der allgemeinen Formel I,

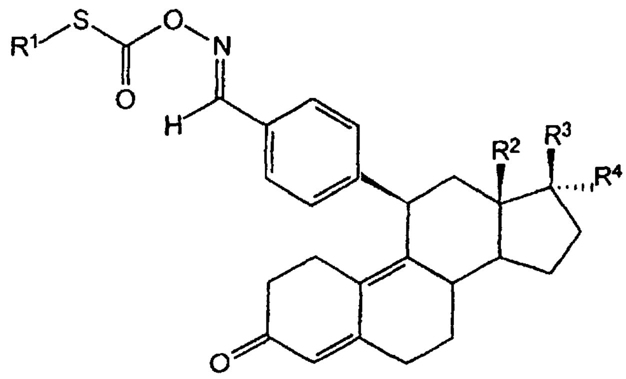

(I)

worin

$R^1$ ein Alkylrest mit 1-10 Kohlenstoffatomen, ein Arylrest mit 6-10 Kohlenstoffatomen oder ein Alkylaryl- oder Arylalkylrest mit jeweils 7-10 Kohlenstoffatomen ist,

$R^2$ für einen Alkylrest mit 1-3 Kohlenstoffatomen oder ein Wasserstoffatom steht,

$R^3$ eine Hydroxylgruppe, eine O-Alkylgruppe mit 1-10 Kohlenstoffatomen, eine O-Arylgruppe mit 6-10 Kohlenstoffatomen, eine O-Aralkyl-, oder O-Alkylarylgruppe mit jeweils 7-10 Kohlenstoffatomen, einen Rest $-OCOR^5$, $-OCONHR^5$ oder $-OCOOR^5$ bedeutet,

wobei

$R^5$ ein Wasserstoffatom, eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen bedeutet,

$R^4$ für ein Wasserstoffatom, eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen steht,

einen Rest $-(CH_2)_nCH_2Y$ bedeutet,

wobei

n = 0, 1 oder 2 ist,

Y für ein Fluor-, Chlor-, Brom- oder Iodatom, für eine Cyano-, Amino-, Azido- oder Rhodanogruppe steht, oder einen Rest $-OR^6$, $-SR^6$, $-(CO)SR^6$ oder $-(CO)OR^6$ bedeutet, wobei

$R^6$ ein Wasserstoffatom, eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen bedeutet, oder ein Rest $-COR^5$ ist, wobei $R^5$ die oben angegebene Bedeutung hat,

einen Rest $-OR^5$ oder $-OCOR^5$ darstellt, wobei $R^5$ die oben angegebene Bedeutung hat,

einen Rest $-(CH_2)_m-CH=CH(CH_2)_p-R^6$ bedeutet,

wobei

m = 0, 1, 2 oder 3 ist,

p = 0, 1 oder 2 bedeutet und

$R^6$ die oben angegebene Bedeutung hat oder einen Rest $-OR^5$ oder $-OCOR^5$ darstellt, wobei $R^5$ die oben angegebene Bedeutung hat,

einen Rest $-(CH_2)_oC{\equiv}CR^7$ darstellt,

wobei

o = 0, 1 oder 2 ist und

$R^7$ für ein Wasserstoffatom, ein Fluor-, Chlor-, Bromoder Iodatom, für eine Alkylgruppe mit 1-10 KohlenStoffatomen, eine Arylgruppe mit 6-10 Kohlenstoffatomen, einen Aralkyl- oder Alkylarylrest mit jeweils 7-10 Kohlenstoffatomen steht oder einen Rest $-OR^5$ darstellt, wobei $R^5$ die oben angegebene Bedeutung hat, oder einen Rest $-OCOR^5$ bedeutet, wobei $R^5$ die oben angegebene Bedeutung hat,

oder einen Rest $-CH_2OR^5$ darstellt, wobei $R^5$ die oben angegebene Bedeutung hat,

oder einen Rest $-C{\equiv}CCH_2OH$ bedeutet,

oder $R^3$ und $R^4$ zusammen einen beliebig substituierten fünf- oder sechsgliedrigen Ring mit mindestens einem Kohlenstoffatom und 0 - 4 Heteroatomen aus der Gruppe Sauerstoff, Schwefel, Selen, Tellur, Stickstoff, Phosphor, Silicium oder Germanium bilden.

Bevorzugt sind Verbindungen, wobei $R^1$ ein Alkylrest mit 1-6 Kohlenstoffatomen ist. Bevorzugt sind auch Verbindungen, worin $R^2$ eine Methyl- oder Ethylgruppe ist. Bevorzugt ist ferner, daß $R^3$ eine Hydroxylgruppe oder eine O-Alkylgruppe mit 1-6 Kohlenstoffatomen darstellt.

Insbesondere bevorzugt sind Verbindungen, bei denen $R^4$ für einen Rest $-OR^5$ oder $-OCOR^5$ steht, wobei $R^5$ einen Alkylrest mit 1-6 Kohlenstoffatomen bedeutet.

Erfindungsgemäß bevorzugt ist ferner, daß $R^4$ für einen Rest $-(CH_2)_m-CH=CH(CH_2)_p-R^6$ steht, wobei

m = 1 und p = 1 ist und

$R^6$ für einen Alkylrest mit 1-6 Kohlenstoffatomen oder

eine Gruppe $-OR^5$ oder $-OCOR^5$ steht, wobei

$R^5$ ein Wasserstoffatom oder eine Alkylgruppe mit 1-6 Kohlenstoffatomen darstellt.

Bevorzugt sind erfindungsgemäß außerdem Verbindungen, bei denen $R^4$ für einen Rest $-(CH_2)_m-CH=CH(CH_2)_p-R^6$ steht, wobei

m = 0 und p = 1 ist und

$R^6$ für eine Gruppe $-OR^5$ oder $-OCOR^5$ steht, wobei

$R^5$ ein Wasserstoffatom oder eine Alkylgruppe mit 1-6 Kohlenstoffatomen ist.

Insbesondere bevorzugt sind auch Verbindungen, bei denen $R^4$ für einen Rest $-(CH_2)_oC{\equiv}CR^7$ steht, wobei

o = 1 ist und

$R^7$ für eine Alkylgruppe mit 1-6 Kohlenstoffatomen steht oder einen Rest $-OCOR^5$ bedeutet, oder für einen Rest $-CH_2OR^5$ steht, wobei

$R^5$ einen Alkylrest mit 1-6 Kohlenstoffatomen oder ein Wasserstoffatom darstellt.

Außerdem sind erfindungsgemäß Verbindungen bevorzugt, bei denen $R^4$ für einen Rest-$(CH_2)_nCH_2Y$ steht, wobei

n = 0 oder 1 ist,

Y für ein F-, Cl-, Br- oder Iodatom, für eine Cyano-,

Amino-, Azido- oder Rhodanogruppe steht, oder ein Rest $-OR^6$ oder $-SR^6$, $-(CO)SR^6$ oder $-(CO)OR^6$ ist, wobei

$R^6$ ein Wasserstoffatom oder eine Alkylgruppe mit 1-10 Kohlenstoffatomen darstellt.

Erfindungsgemäß bevorzugte Verbindungen sind ferner dadurch gekennzeichnet, daß $R^3$ und $R^4$ zusammen einen beliebig substituierten fünf- oder sechsgliedrigen Ring mit mindestens einem Kohlenstoffatom und 0 - 4 Heteroatomen bilden, wobei

die Heteroatome aus der Gruppe Sauerstoff, Schwefel und Stickstoff stammen.

Besonders bevorzugt sind dabei Verbindungen, wobei ein fünfgliedriger Ring ausgebildet wird, der 1 oder 2 Heteroatome enthält.

Ganz besonders bevorzugt sind erfindungsgemäße Verbindungen, wobei der Ring einen Heterocyclus aus der Gruppe Oxazolidinon, Oxazolinon, Thiazolidinon, Thiazolinon, Imidazolidinon, Imidazolinon, 1,3-Dioxolanon, 1,3-Dioxolenon, 1,3-Oxathiolanon, 1,3-Oxathiolenon, Pyrrolidinon, Pyrrolinon, Oxazolidinthion, Oxazolinthion, Thiazolidinthion, Thiazolinthion, Imidazolidinthion, Imidazolinthion, Dioxolanthion, Pyrrolidinthion und Pyrrolinthion darstellt.

Noch bevorzugter sind dabei Verbindungen, wobei der fünfgliedrige Ring ein Oxazolidin-2-on oder ein Oxazolidin-2-thion ist.

Am meisten bevorzugt sind:

4-[17β-Methoxy-17α-(methoxymethyl)-3-oxoestra-4,9-dien-11β-yl]-benzaldehyd-1-(E)-[O-(methylthio)carbonyl] oxim,
4-[17β-Hydroxy-17α-(methoxymethyl)-3-oxoestra-4,9-dien-11β-yl]-benzaldehyd-1-(E)-[O-(methylthio)carbonyl] oxim,
4-[17β-Methoxy-17α-(methoxymethyl)-3-oxoestra-4,9-dien-11β-yl]-benzaldehyd-1-(E)-[O-(ethylthio)carbonyl] oxim,
4-[17β-Methoxy-17α-(methoxymethyl)-3-oxoestra-4,9-dien-11β-yl]-benzaldehyd-1-(Z)-[O-(ethylthio)carbonyl] oxim,
4-[17β-Methoxy-17α-(ethoxymethyl)-3-oxoestra-4,9-dien-11β-yl]-benzaldehyd-1-(E)-[O-(ethylthio)carbonyl]oxim,
4-[17ß-Hydroxy-17α-(methoxymethyl)-3-oxoestra-4,9-dien-11β-yl]-benzaldehyd-1-(E)-[O-(n-propylthio)-carbonyl]oxim,
4-[17β-Methoxy-17α-(n-propoxymethyl)-3-oxoestra-4,9-dien-11β-yl]-benzaldehyd-1-(E)-[O-(methylthio)-carbonyl]oxim, 4-[17β-Hydroxy-17α-(i-propoxymethyl)-3-oxoestra-4,9-dien-11β-yl]-benzaldehyd-1-(E)-[O-(ethylthio)-carbonyl]oxim,
4-[17β-Hydroxy-17α-(methoxymethyl)-3-oxoestra-4,9-dien11β-yl]-benzaldehyd-1-(E)-[O-(ethylthio)-carbonyl) oxim,
4-[17β-Hydroxy-17α-Z-(3-hydroxypropenyl)-3-oxo-estra-4,9-dien-11β-yl]-benzaldehyd-1-(E)-[O-(methylthio)carbonyl]oxim,
4-[17β-Hydroxy-17α-E-(3-hydroxypropenyl)-3-oxo-estra-4,9-dien-11β-yl]-benzaldehyd-1-(E)-[O-(methyl-thio)-carbonyl]oxim,
4-[17β-Methoxy-17α-(3-hydroxy-1-propinyl)-3-oxoestra-4,9-dien-11β-yl]-benzaldehyd-1-(E)-[O-(ethylthio)carbonyl]oxim.
4-[17β-Hydroxy-17α-(azidomethyl)-3-oxoestra-4,9-dien-11β-yl]-benzaldehyd-1-(E)-[O-(ethylthio)-carbonyl]-oxim,
4-[17β-Hydroxy-17α-(chlormethyl)-3-oxoestra-4,9-dien-11β-yl]-benzaldehyd-1-(E)-[O-(ethylthio)carbonyl]-oxim,
4-[17β-Ethoxy-17α-(chlormethyl)-3-oxoestra-4,9-dien-11β-yl]-benzaldehyd-1-(E)-[O-(methylthio)-carbonyl]oxim,
4-[17β-Hydroxy-17α-(cyanomethyl)-3-oxoestra-4,9-dien-11β-yl] -benzaldehyd-1-(E)-[O-(ethylthio)carbonyl]-oxim,
4-[17β-Hydroxy-17α-(ethylthiomethyl)-3-oxoestra-4,9-dien-11β-yl]-benzaldehyd-1-(E)-[O-(ethylthio)-carbonyl] oxim,
4-[17β-Hydroxy-17α-(ethylthiomethyl)-3-oxoestra-4,9-dien-11β-yl]-benzaldehyd-1-(E)-[O-(methylthio)-carbonyl] oxim,
4-[17β-Ethoxy-17α-(methylthiomethyl)-3-oxoestra-4,9-dien-11β-yl]-benzaldehyd-1-(E)-[O-(ethylthio)-carbonyl] oxim,
4-[17β-Hydroxy-17α-[(ethylthiocarbonyl]methyl)-3-oxoestra-4,9-dien-11β-yl]-benz-aldehyd-1-(E)-[O-(ethylthio) carbonyl]oxim,
4-[17β-Hydroxy-17α-(aminomethyl)-3-oxoestra-4,9-dien-11βyl]-benzaldehyd-1-(E)-[O-(ethylthio)carbonyl]-oxim und
(17R)-4-{3-oxoestra-4,9-dien-17-spiro-5'-oxazolidin-2'on-11β-yl)-benzaldehyd-1-(E)-[O-(ethylthio)-carbonyl] oxim.

Gegenstand der vorliegenden Erfindung ist ferner ein Verfahren zur Darstellung der erfindungsgemäßen Verbindungen der allgemeinen Formel 1.

Das erfindungsgemäße Verfahren zur Herstellung von S-substituierten 11β-Benzaldoxim-estra-4,9-dienkohlensäurethiolester der allgemeinen Formel I,

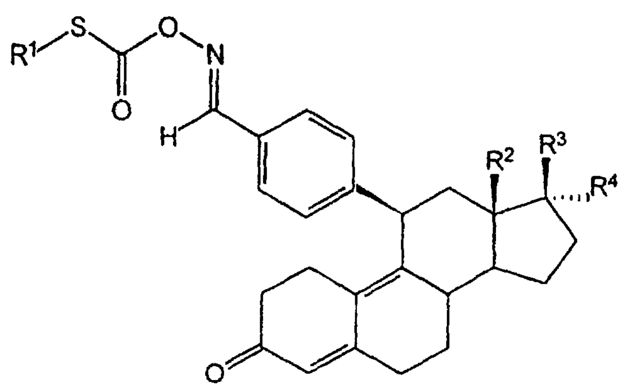

(I)

worin $R^2$, $R^3$ und $R^4$ die oben angegebene Bedeutung haben, ist dadurch gekennzeichnet, daß man in an sich bekannter Weise eine Verbindung der allgemeinen Formel II,

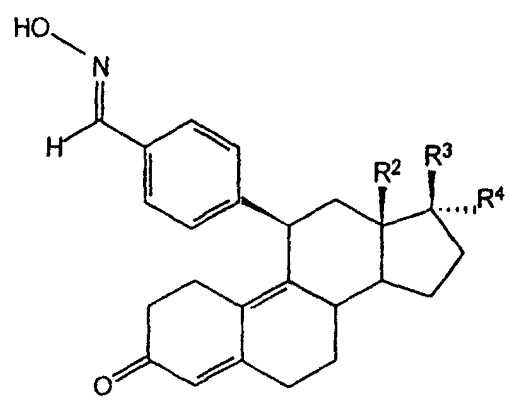

(II)

worin $R^2$, $R^3$ und $R^4$ dieselbe Bedeutung haben wie $R^2$, $R^3$ und $R^4$ in der Formel I, durch Behandlung mit einem Ameisensäurederivat der Formel III

$$Nuc\text{-}(CO)\text{-}SR^1 \qquad (III)$$

worin
$R^1$ die oben genannte Bedeutung hat und Nuc für ein Nucleophil steht,
in einem Lösemittel umsetzt und in eine Verbindung der allgemeinen Formel I überführt.

Bevorzugt ist dabei ein Verfahren, wobei als Lösemittel ein tertiäres Amin einsetzt und die Umsetzung bei einer Temperatur zwischen 20 °C und 80 °C durchführt.

Insbesondere bevorzugt ist ein Verfahren, bei dem man die Umsetzung mit Chlorameisensäurethioestern in Pyridin oder Triethylamin bei einer Temperatur zwischen 20 °C und 40 °C durchführt.

Die Herstellung der Ausgangsverbindungen der allgemeinen Formel II erfolgt, wenn nicht anders angegeben ist, nach den Vorschriften der Schriften EP-A-0 648 778 oder EP-A-0 648 779.

Die Herstellung der pharmazeutisch annehmbaren Salz erfolgt in ans ich bekannter Weise. Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beispielsweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wäßrige Lösungen von Säureadditionssalzen der Verbindung der Formel I in einer wäßrigen Säurelösung hergestellt werden.

Die Säureadditionssalze der Verbindungen der allgemeinen Formel I können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Ein weiterer Gegenstand der vorliegenden Erfindung sind pharmazeutische Zusammensetzungen, gekennzeichnet durch den Gehalt an mindestens einer Verbindung der allgemeinen Formel I.

Gegenstand der vorliegenden Erfindung sind auch Arzneistoffe zur oralen, rektalen, subcutanen, intravenösen oder intramuskulären Anwendung, die zusammen mit den üblichen Träger- und Verdünnungsmitteln mindestens eine Verbindung der allgemeinen Formel I als Wirkstoff enthalten.

Die Arzneimittel der Erfindung werden mit den üblichen festen oder flüssigen Trägerstoffen und/oder Verdünnungsmitteln und den üblicherweise verwendeten pharmazeutischtechnischen Hilfsstoffen entsprechend der gewünschten Applikationsart in einer geeigneten Dosierung und in an sich bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffes mit bekannten Hilfsstoffen, beispielsweise Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid, oder Zucker hergestellt werden. Die Drageehülle kann dabei auch aus mehreren Schichten bestehen,
wobei beispielsweise die oben bei den Tabletten genannten Hilfsstoffe verwendet werden können.

Die Lösungen oder Suspensionen mit dem erfindungsgemäßen Wirkstoff können zur Verbesserung des Geschmacks mit Stoffen wie Saccharin, Cyclamat oder Zucker und/oder mit Aromastoffen, wie Vanillin oder Orangenextrakt versetzt werden. Weiterhin können sie mit Suspendierhilfsstoffen wie Natriumcarboxymethylcellulose oder Konservierungsmitteln wie p-Hydroxybenzoesäure vermischt werden.

Die Bereitung von Kapseln kann durch Mischen des Arzneistoffes mit Trägern wie Milchzucker oder Sorbit erfolgen, die dann in die Kapseln eingebracht werden.

Die Herstellung von Suppositorien erfolgt beispielsweise durch Mischung des Wirkstoffes mit geeigneten Trägermaterialien wie Neutralfetten oder Polyethylenglykolen oder dessen Derivaten.

Die erfindungsgemäßen Verbindungen der allgemeinen Formel I werden am Progesteronrezeptor gebunden (vgl. Tabelle 1) und besitzen im Vergleich zu RU 486 eine deutlich reduzierte antiglucocorticoide Wirkung, nachgewiesen durch die verminderte Glucocorticoid-Rezeptorbindung *in vitro* (vgl. Tabelle 1).

Tabelle 1

| Rezeptorbindung von S-substituierten 11β-Benzaldoximestra-4,9-dien-kohlensäurethiolestern | | |
|---|---|---|
| Verbindung nach Beispiel | relative molare Bindungsaffinität RBA (%) zum Progesteron-rezeptor Progesteron = 100 % | relative molare Bindungsaffinität RBA (%) zum Glucocorticoid-rezeptor Dexamethason = 100 % |
| 1 (J 1241) | 159 | 49 |
| 2 (J 1247) | 185 | 52 |
| 3 (J 1042) | 164 | 42 |
| 4 (J 1234) | 144 | 53 |
| 5 (J 1240) | 77 | 20 |
| 6 (J 1245) | 64 | 22 |
| 7 (J 1230) | 42 | 2 |
| 8 (J 1244) | 74 | 32 |
| zum Vergleich: | | |
| RU 486 (Mifepriston) | 506 | 685 |
| J 867 | 302 | 77 |
| ZK 98299 (Onapriston) | 22 | 39 |

J 867 = 11β-[4-(Hydroximinomethyl)phenyl]-17β-Methoxy-17α-methoxymethyl-estra-4,9-dien-3-on (EP-A-0 648 778 und EP-A-0 648 779)

Tabelle 2

| Frühabortive Wirkung bei der Ratte nach subcutaner Applikation vom 5.-7.-Graviditätstag (Applikation 0,2 ml/(Tier*Tag) in Benzoylbenzoat/Rizinusöl (1+4 v/v)) | | | |
|---|---|---|---|
| Verbindung nach Beispiel | Dosis (mg/(Tier*Tag)) | komplette Graviditätshemmung* | |
| | | $N^{\#}/N$ | % |
| Vehikel | | 0/6 | 0 |
| 2 (J 1247) | 3 | 0/4 | 0 |
| 3 (J 1042) | 10 | 1/5 | 20 |
| | 3 | 0/5 | 0 |
| | 1 | 0/5 | 0 |
| 7 (J 1230) | 3 | 0/5 | 0 |
| | 1 | 0/5 | 0 |

* leere Uteri
N Zahl der angepaarten Weibchen
$N^{\#}$ Zahl der nichtgraviden Weibchen

Tabelle 2 (fortgesetzt)

| Frühabortive Wirkung bei der Ratte nach subcutaner Applikation vom 5.-7.-Graviditätstag (Applikation 0,2 ml/(Tier*Tag) in Benzoylbenzoat/Rizinusöl (1+4 v/v)) | | | |
|---|---|---|---|
| Verbindung nach Beispiel | Dosis (mg/(Tier*Tag)) | komplette Graviditätshemmung* | |
| | | $N^{\#}/N$ | % |
| RU 486 (Vergleich) | 3 | 5/5 | 100 |
| | 1 | 1/5 | 20 |
| | 0,3 | 0/5 | 0 |
| J 867 (Vergleich) | 3 | 5/5 | 100 |
| | 1 | 5/5 | 100 |
| | 0,3 | 0/5 | 0 |

* leere Uteri
N Zahl der angepaarten Weibchen
$N^{\#}$ Zahl der nichtgraviden Weibchen

Überraschenderweise wurde gefunden, daß erfindungsgemäße Substanzen, die eine sehr hohe Affinität zum Progesteronrezeptor besaßen (siehe Tabelle 1), nicht oder nur bei sehr hohen Dosierungen in der Lage waren die frühe Gravidität bei Ratten zu stören. Die erfindungsgemäßen Substanzen (wie beispielsweise J 1042) erwiesen sich auch bei höchsten Dosierungen als unfähig die Gravidität von Meerschweinchen zu stören (siehe Tabelle 3).

Tabelle 3

| Abortive Wirkung am Meerschwein nach subcutaner Applikation am 43.-44. Graviditätstag | | | |
|---|---|---|---|
| Substanz nach Beispiel | Dosis mg/Tier/Tag | komplette Graviditätshemmung | |
| | | $N^{\#}/N$ | % |
| 3 (J 1042) | 10 | 0/7 | 0 |
| | 30 | 0/7 | 0 |
| | 100 | 0/7 | 0 |
| N Zahl der angepaarten Weibchen<br>$N^{\#}$ Zahl der nichtgraviden Weibchen | | | |

Die erfindungsgemäßen Verbindungen haben trotz hoher Rezeptoraffinität keine Hemmung der Luteolyse zur Folge. Überraschend ist nun, daß die Verbindungen der Formel I antiovulatorische und progesteronanaloge Aktivität beim Meerschweinchen besitzen. Anders als unter Onapriston, das beim zyklischen Meerschweinchen trotz hoher Progesteronspiegel im Blut Proliferation und Verhornung des Vaginalepithels induziert, wird unter den erfindungsgemäßen Verbindungen (trotz niedriger Progesteronspiegel im Blut) eine vollständige Hemmung der Proliferation dieses Epithels und Muzifizierung als Ausdruck einer Progesterondominanz gesehen. In dieser Struktur entspricht der Effekt der erfindungsgemäßen Substanzen demjenigen von mitgeprüften Gestagenen (Progesteron bzw Levonorgestrel).

Im Hinblick auf andere Parameter beim Meerschweinchen lassen sich die erfindungsgemäßen Verbindungen der Formel I sowohl gegen "reine" Antagonisten des Typs I (Onapriston) als auch gegen Agonisten (Progesteron) abgrenzen. Onapriston führt zu sehr niedrigen Prostaglandinspiegeln im Blut, Progesteron und Levonorgestrel dagegen zu einer erhöhten und verlängerten uterinen Sekretion von $PGF_{2\alpha}$, reflektiert durch erhöhte Prostaglandin F-Metabolitspiegel (PGMF-Spiegel) im Blut. PGFM ist der langlebige Hauptmetabolit des vom Endometrium gebildeten $PGF_{2\alpha}$. Die erfindungsgemäßen Verbindungen führen zu erniedrigten PGFM-Spiegeln im Vergleich zu zyklischen Kontrolltieren in der Phase der Luteolyse und im Vergleich zu mit Gestagen behandelten Tieren. Die PGFM-Spiegel liegen aber nicht so tief wie bei mit Onapriston behandelten Tieren.

Im Kaninchen haben die erfindungsgemäßen Substanzen transformatorische Aktivität im McPhail-Test und überraschenderweise antitransformatorische Aktivität im gleichen Test bei Kombination mit Progesteron.

Tierexperimentelle Untersuchungen zeigen, daß die erfindungsgemäßen Progesteronantagonisten so starke partialagonistische Wirkungen am Progesteronrezeptor besitzen, daß abortive Effekte nicht mehr in Erscheinug treten. Überraschenderweise werden therapierelevante Eigenschaften wie beispielsweise die Hemmung der uterinen Prostaglandinsekretion, die Hemmung proliferativer Vorgänge in Geweben des Genitaltraktes und antiovulatorische Eigenschaften dagegen weiterhin aufgefunden.

Für den hier beschriebenen Wirkstofftyp wird die Bezeichnung"*Mesoprogestin*" vorgeschlagen, da die Bezeichnung Antigestagen definitionsgemäß auch abortive Eigenschaften impliziert, die bei den erfindungsgemäßen Verbindungen tierexperimentell nicht nachgewiesen werden können.

Die erfindungsgemäßen Verbindungen sind hochaffine, hochselektive Modulatoren der Steroidrezeptoren. Insbesondere sind sie Agonisten oder Antagonisten der Progesteron- und Androgenrezeptoren.

Ein weiterer Gegenstand der vorliegenden Erfindung ist daher die Verwendung der erfindungsgemäßen Verbindungen der allgemeinen Formel I für die Behandlung von Endometriose, Uterus myomatosus, Dysmennorrhoe und praemenstruellem Syndrom, zur Induktion einer reversiblen Amennorhoe ohne Oestrogendefizit und für die klimakterische Substitutionstherapie (Hormone Replacement Therapy HRT) gegebenenfalls in Kombination mit Oestrogenen. Erfindungsgemäß ist auch die Verwendung zur Herstellung von Kontrazeptiva.

Die erfindungsgemäßen Verbindungen der allgemeinen Formel I sind erfindungsgemäß auch bei folgenden Indikationen verwendbar, wie dysfunktionale uterine Blutungen, Hämorrhagie, Fertilitätskontrolle und Fertilitätsmodulation, Myom, Leiomyom, Osteoporose, Akne, Tumore wie Brusttumore, endometrielle Tumore, ovarielle Tumore, Endometriose, prostatische Hyperplasie, Prostatatumore, hormonell bedingte Glatzenbildung und androgene Erkrankungen und Ausfallerscheinungen.

Die nachfolgenden Beispiele erläutern die Erfindung:

Allgemeine Vorschrift zur Synthese der S-substituierten 11β-Benzaldoxim-estra-4,9-dien-Derivate

Zu (2 mmol) 4-[17β-substituiertes-17α-substituiertes-3-oxoestra-4,9-dien-11β-yl]-benzaldehyd-(E oder Z) oxim in 10 ml Pyridin werden 3 mmol des entsprechenden Chlorameisensäure-S-alkyl(aryl)-ester zugegeben. Man rührt bei Raumtemperatur bis zum vollständigen Umsatz, gießt in Wasser ein und saugt den ausfallenden Niederschlag ab, wäscht mit Wasser und trocknet. Das Rohprodukt wird zur Reinigung chromatographiert und/oder umkristallisiert.

Beispiel 1

4- [17β-Methoxy-17α-(methoxymethyl)-3-oxoestra-4,9-dien-11β-yl]-benzaldehyd-1-(E)-[O-(methylthio)carbonyl]oxim,

Ausbeute: 71 % d.Th.
Schmp. 134 -137 °C (Aceton / Methyl-tert.butylether);
$\alpha_D$ = + 184 ° ($CHCl_3$) ;
IR in KBr [$cm^{-1}$]: 1657 (C=C-C=C-C=O) ; 1740 ($-OC=OSCH_3$) ; UV [MeOH] : $\lambda_{max}$ 273 nm; ε = 24 505, log ε = 4.39, $\lambda_{max}$ = 289 nm, ε = 22 690, log ε = 4.36.
$^1$H-NMR: [$CDCl_3$; TMS] (δ, ppm) : 0,52 (s, 3H, H-18) ; 2,40 (s, 3H, $SCH_3$) ; 3,25 (s, 3H , $OCH_3$) ; 3,40 (s, 3H, $OCH_3$) ; 3,42 und 3,57 (2d, 2H, J = 10.5 Hz, 17α-$CH_2OCH_3$) ; 4,41 (d, 1H, J = 6,9 Hz, H-11α); 5,78 (s, 1H, H-4); 7,29 (d, 2H, J = 4,5 Hz, H-2' ); 7,61 (d, 2H, J = 7,8 Hz, H-3'); 8,32 (s, 1H CH=N-OC=$OSCH_3$)

Beispiel 2

4-[17β-Hydroxy-17α-(methoxymethyl)-3-oxoestra-4,9-dien-11β-yl]-benzaldehyd-1-(E)-[O-(methylthio)carbonyl]oxim,

Ausbeute: 68 % d. Th
Schmp. 186-189 °C (Aceton);
$\alpha_D$ = + 230 ° ($CHCl_3$) ;
IR in KBr [$cm^{-1}$]: 1602, 1652 (R-CO-R, Ph, >C=N-) ; 1729 (-S-CO-O-) ;
UV [MeOH] : $\lambda_{max}$ 274 nm; ε = 30780, log ε = 4,49, $\lambda_{max}$ = 289 nm, ε = 28560, log ε = 4,55.
$^1$H-NMR [$CDCl_3$; TMS] (δ, ppm) : 0,52 (s, 3H, H-18) 2,40 (s, 3H, $-SCH_3$) 3,21 (d, 1H, J = 8,8 Hz, $-CH_2$-O-) 3,41 (s, 3H, $-O-CH_3$) 3,56 (d, 1H, J = 8,8 Hz, $-CH_2$-O-) 4,42 (d, 1H, J = 7,1 Hz, H-11) 5,79 (s, 1H, H-4) 7,28 (d, 2H, J = 8,1 Hz, H-3') 7,62 (d, 2H, J = 8,1 Hz, H-2') 8,32 (s, 1H, - HC=N)

Beispiel 3

4-[17β-Methoxy-17α-(methoxymethyl)-3-oxoestra-4,9-dien-11β-yl]-benzaldehyd-1-(E)-[O-(ethylthio)carbonyl]oxim,

Ausbeute: 70 % d.Th.
Schmp.: 148 - 155°C (Aceton/Hexan) ;
$\alpha_D$ = + 235° ($CHCl_3$),
IR in KBr [$cm^{-1}$] : 1606 (Phenyl), 1653 (C=C-C=C-C=O), 1745 (-OC=OSEt),

UV (MeOH) : $\lambda_{max}$ = 274 nm ε = 31 085, log ε = 4.49, $\lambda_{max}$ = 298 nm, ε = 28 280, log ε = 4.45.
$^1$H-NMR[$CDCl_3$; TMS] (δ, ppm): 0,52 (s, 3H, H-18); 1,37 (t, 3H, J = 7,5 Hz, $SCH_2CH_3$) ; 2,95 (q, 2H, J = 4,5 und 15 Hz, $SCH_2CH_3$) ; 3,25 (s, 3H, $OCH_3$) ; 3,41 (s, 3H, $OCH_3$); 3,42 und 3,57 (2d, 2H, J = 10,5 Hz und 10,8 Hz, $CH_2O$) ; 4,41 (d, 1H, J = 7,2 Hz, H-11α) ; 5,79 (s, 1H, H-4) ; 7,27 (d, 2H, J = 8,4 Hz, H-2') ; 7,61 (d, 2 H, J = 8,4 Hz, H-3') ; 7,54 (s, 1H, OH); 8,31 (s, 1H, CH=NOCOSEt)

Beispiel 4

4-[17β-Hydroxy-17α-(methoxymethyl)-3-oxoestra-4,9-dien-11β-yl]-benzaldehyd-1-(E)-[O-(ethylthio)carbonyl]oxim,

Ausbeute: 74 % d. Th.
Schmp. 176-180 °C (Dichlormethan/Essigester) ;
$\alpha_D$ = + 226° ($CHCl_3$) ;
IR in KBr [$cm^{-1}$] : 1600, 1653 (R-CO-R, Ph, >C=N-) 1729, (-S-CO-O- C=O) ;
UV [MeOH] : $\lambda_{max}$ 274 nm; ε = 31140, log ε = 4,50, $\lambda_{max}$ = 289 nm, ε = 28720, log ε = 4,46.
$^1$H-NMR [$CDCl_3$; TMS] (δ, ppm) : 0,52 (s,3H, H-18) 1,36 (t,3H, J = 7,2 Hz, $-CH_2CH_3$) 2,73 (dt, 2H, J = 1,5, 5,3 Hz, H-7) 2,95 (q, 2H, J = 7,2 Hz, $-CH_2CH_3$) 3,21(d, 1H, J = 9,2 Hz, $-CH_2$-O-) 3,42(s, 3H, $-O-CH_3$) 3,56 (d, 1H, J = 9,2 Hz, $-CH_2$-O-) 4,41 (d, 1H, J = 6,7 Hz, H-11) 5,79 (s, 1H, H-4) 7,27 (d, 2H, J = 8,2 Hz, H-3') 7,62 (d, 2H, J = 8,2 Hz, H-2') 8,32 (s, 1H, -HC=N)

Beispiel 5

4-[17β-Hydroxy-17α-(azidomethyl)-3-oxoestra-4,9-dien-11βyl]-benzaldehyd-1-(E)-[O-(ethylthio)carbonyl]oxim,

Ausbeute: 64 % d.Th.
Schmp. 168 - 171 °C (Aceton);
$\alpha_D$ = + 197° ($CHCl_3$) ;
IR in KBr [$cm^{-1}$] : 1658 (C=C-C=C-C=O) ; 1693,1715 (-OC=OSEt), 2094 ($N_3$),
UV [MeOH] : $\lambda_{max}$ 274 nm ε = 33 150, log ε = 4,49, $\lambda_{max}$ = 288 nm, ε = 28 140, log ε = 4,45.
$^1$H-NMR: [$CDCl_3$; TMS] (δ, ppm): 0,54 (s, 3H, H-18) ; 1,37 (t, 3H, J = 7,2 Hz, $SCH_2CH_3$) ; 1,61 (s, 1 H, OH); 2,95 (q, 2H, J = 4,5 und 14,7 Hz $CH_2CH_3$) ; 3,30 und 3,59 (2d, 2H, J = 12 Hz, 17α-$CH_2N_3$) ; 4,45 (d, 1H, J = 6,9 Hz, H-11α) ; 5,80 (s, 1H, H-4); 7,29 (d, 2H, J = 8,4 Hz, H-2' ); 7,64 (d, 2H, J = 8,4 Hz, H-3'); 8,32 (s, 1H CH=N-OC=$OSC_2H_5$ )

Herstellung der Ausgangsverbindung

Stufe A

1g 4-[3,3-Dimethoxy-5α-hydroxy-17(S)-spiroepoxy-estr-9-en-11β-yl]benzaldehyd-1-ethylenketal werden in 50 ml Ethylenglykol suspendiert und mit 1g Natriumazid bei 100°C 2,5 h gerührt. Die abgekühlte Lösung wird in Wasser eingerührt, der Niederschlag wird abgesaugt, neutral gewaschen und getrocknet. Man erhält 870 mg 4-[17α-Azidomethyl-3,3-dimethoxy-5α,17β-dihydroxy-estr-9-en-11β-yl]benzaldehyd-1-ethylenketal als hellbraunen Schaum, der direkt in der nächste Stufe eingesetzt wird.
$^1$H-NMR [$CDCl_3$; TMS]: 0,47 (s, 3H, H-18); 1,87 (s, 1H, OH); 3,21 und 3,23 (2s, je 3H, $OCH_3$) ; 3,22 und 3,54 (2d, 2H, J = 10,8 Hz, $CH_2N_3$) ; 4,0-4,16 (2m, 4 H, Ethylenketal); 4,29 (d, 1H, J = 7,2 Hz, H-11α); 4,68 (s, 1H OH); 5,76 (s, 1H, CH-ketal) ; 7,23 (d, 2H, J = 8,4 Hz, H-2') ; 7,38 (d, 2 H, J = 8,4 Hz, H-3').

Stufe B

650 mg 4-[17α-Azidomethyl-3,3-dimethoxy-5α,17β-dihydroxyestr-9-en-11β-yl]benzaldehyd-1-ethylenketal werden in 12 ml Aceton und 1,2 ml Wasser mit 155 mg p-Toluolsulfonsäure bei Raumtemperatur umgesetzt. Nach 2 h wird mit wäßrigem Ammoniak neutralisiert, wobei ein Niederschlag ausfällt, der abgesaugt und getrocknet wird. Die Umkristallisation von 4-[17α-Azidomethyl-17β-hydroxy-3-oxoestra-4,9-dien-11β-yl]benzaldehyd erfolgt aus Aceton.
Schmp.: 197-205 °C (Aceton);
$\alpha_D$ = + 156 ° ($CHCl_3$) ;
IR (KBr) [$cm^{-1}$] : 1648 (C=C-C=C-C=O) ; 1712 (CHO), 2100 ($N_3$) ; UV [MeOH] : $\lambda_{max}$ 203 nm, ε = 21 143, log ε = 4,32; $\lambda_{max}$ 263 nm, ε = 8338, log ε = 4,26; $\lambda_{max}$ 299 nm, ε = 20 712, log ε = 4,32 ;
$^1$H-NMR: [$CDCl_3$; TMS] (δ, ppm): 0,53 (s, 3H, H-18); 2,04 (s, 1H, OH); 3,31 und 3,60 (2d, 2H, J = 12,3 Hz, 17α-$CH_2N_3$); 4,48 (d, 1H, J = 7,2 Hz, H-11α) 5,81 (s, 1H, H-4); 7,37 (d, 2H, J = 8,4 Hz, H -2') ; 7,82 (d, 2H, J = 8,4 Hz, H -3') ; 9,98

(s, 1H CHO).

Stufe C

495 mg 4-[17α-Azidomethyl-17β-hydroxy-3-oxoestra-4,9-dien-11β-yl]benzaldehyd werden in 5 ml Pyridin und 80 mg Hydroxylaminhydrochlorid innerhalb von 3h bei Raumtemperatur umgesetzt. Man gießt in Eiswasser ein und saugt den farblosen Niederschlag ab, trocknet und reinigt durch Chromatographie. Man erhält 380 mg 4-[17α-Azidomethyl-17β-hydroxy-3-oxoestra-4,9-dien-11β-yl]benzaldehyd(E)-oxim.
Schmp.: 145 - 151 und 193 -200 °C (Methyl-tert.butylether);
$\alpha_D$ = + 212 ° ($CHCl_3$) ;
IR (KBr) [$cm^{-1}$] : 1643, 1657 (C=C-C=C-C=O) ; 2099 ($N_3$) ;
UV [MeOH] : $\lambda_{max}$ 265 nm, ε = 21 765, logε = 4,34, $\lambda_{max}$ 299 nm, ε = 22 520, log ε = 4,35;
$^1$H-NMR: [DMSO; TMS] (δ, ppm): 0,43 (s, 3H, H-18); 2,04 (s, 1H, OH); 3,09 und 3,40 (2d, 2H, J = 12,0 Hz, 17α-$CH_2N_3$); 4,40 (d, 1H, J = 6,2 Hz, H-11α); 4,74 (s, 1H, OH); 5,68 (s, 1H, H-4); 7,24 (d, 2H, J = 8,1 Hz, H -2); und 7,51 (d, 2H, J=8,1 Hz, H-3') ; 8,10 (s, 1H, CH=NOH) ;
11,16 (2, 1H, OH).

Beispiel 6

4-[17β-Hydroxy-17α-(chlormethyl)-3-oxoestra-4,9-dien-11β-yl]-benzaldehyd-1-(E)-[O-(ethylthio)carbonyl]oxim

Schmp.: 164 - 169°C (Aceton/Methyl-tert.butylether);
$\alpha_D$ = + 222° ($CHCl_3$) ;
IR in KBr [$cm^{-1}$] : 1595 (Phenyl), 1643 (C=C-C=C-C=O), 1740 (-OC=OSEt);
UV [MeOH] : $\lambda_{max}$ 273 nm; ε = 30 950, log ε = 4,49, $\lambda_{max}$ = 288 nm, ε = 28 140, log ε = 4,45.
$^1$H-NMR-[$CDCl_3$; TMS] 0,59 (s, 3H, H-18) ; 1,37 (t, 3H, J = 7,5 Hz, $SCH_2CH_3$) ; 2,95 (q, 2H, J = 4,5 und 15 Hz, $SCH_2CH_3$) ; 3,65, 3,84 (2d, 2H, J = 10,8 Hz und 11,1 Hz, $CH_2O$) ; 4,43 (d, 1H, J = 7,2 Hz, H-11α) ; 5,80 (s, 1H, H-4); 7,27 (d, 2H, J = 8,1 Hz, H-2') ; 7,64 (d, 2 H, J = 8,4 Hz, H-3') ; 8,32 (s, 1H, CH=NOCOSEt) ;

Beispiel 7

4- [17β-Hydroxy-17α-(cyanomethyl)-3-oxoestra-4, 9-dien-11βyl]-benzaldehyd-1-(E)-[O-(ethylthio)carbonyl]oxim

Ausbeute: 75 % d. Th
Schmp. 178-181 °C (Aceton/ Methyltertiärbutylether) ;
$\alpha_D$ = + 222 ° ($CHCl_3$) ;
IR in KBr [$cm^{-1}$] : 1595, 1637 (R-CO-R, Ph, >C=N-) 1736
(-S-CO-O-); 2247 (-$CH_2$-CN)
UV [MeOH] : $\lambda_{max}$ 273 nm; ε = 31310, log ε = 4,50, $\lambda_{max}$ = 288 nm, ε = 28560, log ε = 4,55.
$^1$H-NMR [$CDCl_3$; TMS]: 0,57 (s,3H, H-18) 1,36 (t, 3H, J = 7,5 Hz, -$CH_2CH_3$) 2,95 (q, 2H, J = 7,5 Hz, -$CH_2CH_3$) 4,48 (d, 1H, J = 6,7 Hz, H-11) 5,80 (s, 1H, H-4) 7,27 (d, 2H, J = 8,2 Hz, H-3') 7,64 (d, 2H, J = 8,2 Hz, H-2')

Beispiel 8

4-{17β-Hydroxy-17α-[(ethylthiocarbonyloxy)methyl]3-oxoestra-4,9-dien-11β-yl}-benzaldehyd-1-(E)-[O-(ethylthio)carbonyl]oxim,

Ausbeute: 53 % d.Th.
Schmp. 152 - 156°C (Aceton);
$\alpha_D$ = + 166 ° ($CHCl_3$) ;
IR in KBr [$cm^{-1}$] : 1655 (C=C-C=C-C=O) ; 1691 (-OC=OSEt) ;
UV [MeOH] : $\lambda_{max}$ 273 nm; ε = 31 290, log ε = 4,50, $\lambda_{max}$ = 288 nm, ε = 28 490, log ε = 4,45.
$^1$H-NMR: [$CDCl_3$; TMS] 0,55 (s, 3H, H-18); 1,35 und 1,37 (2t, 2x 3H, $CH_2CH_3$) ; 2,13 (s, 1H, OH) ; 2,40 (s, 3H, $SCH_3$) ; 2,87-2,99 (m, 4H, 2x $CH_2CH_3$) ; 4,20 und 4,34 (2d, 2H, J = 10,8 Hz, 17α-$CH_2OCH_3$) ; 4,43 (d, 1H, J= 6,9 Hz, H-11α) ; 5,80 (s, 1H, H-4); 7,27 (d, 2H, J = 8,1 Hz, H-2'); 7,64 (d, 2H, J = 8,4 Hz, H-3') ; 8,32 (s, 1H CH=N-OC=OSCH$_3$)

Herstellung der Ausgangsverbindung

Stufe A

3 g 4-[3,3-Dimethoxy-5α-hydroxy-17(S)-spiroepoxy-estr-9-en-11β-yl]benzaldehyd-1-ethylenketal wird in 65 ml Methylpyrrolidon mit 22 ml 2N NaOH 5 h auf 110 °C erhitzt und anschließend in Eiswasser eingegossen. Es wird mit Essigester extrahiert, die organische Phase neutral gewaschen, getrocknet und unter Vakuum verdampft.

Das dunkle Öl wird durch Chromatographie gereinigt. Man erhält 1,13 g mg 4-[3,3-Dimethoxy-5α,17β-dihydroxy-17α-(hydroxymethyl)-estr-9-en-11β-yl]benzaldehyd-1-ethylenketal als blaßgelben Schaum, der direkt in die nächste Stufe eingesetzt wird.

$^1$H-NMR [$CDCl_3$; TMS] 0,47 (s, 3H, H-18) ; 2,04 (s, 1H, OH); 3,21 und 3,22 (2s, je 3H, $OCH_3$) ; 3,40 und 3,74 (2d, 2H, J = 10,8 Hz, $CH_2OH$ nach HD-Austausch) ; 4,0-4,15 (2m, 4 H, Ethylenketal); 4,29 (d, 1H, J = 7,2 Hz, H-11α) ; 4,67 (s, 1H OH); 5,76 (s, 1H, CH-ketal); 7,23 (d, 2H, J = 8,4 Hz, H-2') ; 7,37 (d, 2 H, J = 8,4 Hz, H-3').

Stufe B

1,13 g 4-[3,3-Dimethoxy-5α,17β-dihydroxy-17α-(hydroxymethyl)-estr-9-en-11β-yl]-benzaldehyd-1-ethylenketal werden in 17 ml THF gelöst und mit 2,0 ml Wasser und 260 mg p-Toluolsulfonsäure 4 h bei Raumtemperatur gerührt. Unter Vakuum wird auf das halbe Volumen eingeengt und die Lösung in Eiswasser eingerührt. Es wird mit Essigester 2x extrahiert, die organische Phase neutral gewaschen, getrocknet und unter Vakuum verdampft. Das Rohprodukt wird mehrmals aus Essigester umkristallisiert. Man erhält 308 mg 4-[17β-Hydroxy-17α-(hydroxymethyl)-3-oxoestra-4,9-dien-11β-yl]benzaldehyd.

Schmp.: 211 - 220°C (Essigester);

$\alpha_D$ = + 185° ($CHCl_3$) ;

IR in KBr [$cm^{-1}$] : 1661 (C=C-C=C-C=O), 1693 (CH=O) ;

UV [MeOH] : $\lambda_{max}$ 264 nm; ε = 14 560, log ε = 4.16, $\lambda_{max}$ = 299 nm, ε = 16 180, log ε = 4.20.

$^1$H-NMR [$CDCl_3$; TMS] 0,53 (s, 3H, H-18); 3,4 und 3,8 (2m, 2H, $CH_2O$, 4,65 d und 4,95 d nach TAI-Zugabe: J = 12,0 Hz); 4,43 (d, 1H, J = 7,2 Hz, H-11α) ; 5,80 (s, 1H, H-4); 7,38 (d, 2H, J = 8,1 Hz, H-2') ; 7,81 (d, 2 H, J = 8,4 Hz, H-3') ; 9,97 (s, 1H, CH=O).

Stufe C

752 mg 4-[17β-Hydroxy-17α-(hydroxymethyl)-3-oxoestra-4,9-dien-11β-yl]benzaldehyd werden in 8 ml Pyridin unter Argon mit 128 mg Hydroxylaminhydrochlorid innerhalb von 40 Minuten bei Raumtemperatur unter Rührung umgesetzt. Die Lösung wird in Eiswasser eingegossen, der Niederschlag wird abgesaugt, gewaschen und getrocknet. Man erhält 690 mg 4-[17β-Hydroxy-17α-(hydroxymethyl)-3-oxoestra-4,9-dien-11β-yl]benzaldehyd-(E)oxim.

Schmp.: 198-204 °C (Essigester/Methyl-tert.butylether);

$\alpha_D$ = + 237° (MeOH) ;

IR in KBr [$cm^{-1}$]: 1637, 1650, 1657 (C=C-C=C-C=O);

UV [MeOH] : $\lambda_{max}$ 264 nm; ε = 20 503, log ε = 4.31, $\lambda_{max}$ = 299 nm, ε = 20 020, log ε = 4.30;

$^1$H-NMR [$CDCl_3$; TMS]: 0,53 (s, 3H, H-18) ; 3,4 und 3,8 (2m, 2H, $CH_2O$, 4,65 d und 4,95 d nach TAI-Zugabe: J = 12,0 Hz); 4,43 (d, 1H, J = 7,2 Hz, H-11α); 5,80 (s, 1H, H-4); 7,38 (d, 2H, J = 8,1 Hz, H-2'); 7,81 (d, 2 H, J = 8,4 Hz, H-3') ; 9,97 (s, 1H, CH=O).

Beispiel 9

4-[17β-Hydroxy-17α-(ethylthiomethyl)-3-oxoestra-4,9-dien-11β-yl]-benzaldehyd-1-(E)-[O-(ethylthio)carbonyl]oxim

Ausbeute: 73 % d.Th.

Schmp.: 138 - 141 °C (Aceton/EtOH) ;

$\alpha_D$ = + 184 ° ($CHCl_3$) ;

IR in KBr [$cm^{-1}$] : 1602 (Phenyl), 1646, 1650 (C=C-C=C-C=O) 1731,1737 (OC=OSEt);

UV [MeOH] : $\lambda_{max}$ 274 nm ε = 31 420, log ε= 4.50, $\lambda_{max}$ 288 nm ε = 28 750, log ε = 4.46; $\lambda_{max}$ 297 nm ε = 28 220, log ε = 4.45;

$^1$H-NMR: [$CDCl_3$; TMS] : 0,55 (s, 3H, H-18) ; 1,29 (t 3H, J = 7,2 Hz, $SCH_2CH_3$) ; 1,37 (t, 3H, J= 7,2 Hz, $SCH_2CH_3$) ; 2,60 (q, 2H; J= 7,2 Hz und J = 14,7 Hz, $SCH_2CH_3$) ; 2,71 und 2,96 (2d, 2H, J = 12,9 Hz, 17α-$CH_2SC_2CH_3$) ; 2,85 (s, 1H, OH); 2,95 (m, 2H, $SCH_2CH_3$) ; 4,44 (d, 1H, J = 7,2 Hz, H-11α) ; 5,79 (s, 1H, H-4) ; 7,27 (d, 2H, J = 8,4 Hz, H-2') ; 7,63 (d, 2H, J = 8,4 Hz, H-3') ; 8,32 (s, 1H CH=NOR).

Herstellung der Auscrangsverbindung

Stufe A

1,48 g 4-[3,3-Dimethoxy-5α-hydroxy-17(S)-spiroepoxyestr-9-en-11β-yl]benzaldehyd-1-ethylenketal wird mit 500 mg Natriumthioethanolat in 15 ml DMSO unter Rührung 2h auf 80 °C erwärmt. Man gießt in Eiswasser ein, saugt ab und wäscht neutral. Nach Trocknung werden 1,47 g 4-[3,3-Dimethoxy-5α,17β-dihydroxy-17α-(ethylthiomethyl)-estr-9-en-11β-yl]benzaldehyd-1-ethylenketal als braunes Rohprodukt erhalten, das direkt in die nächste Stufe eingesetzt wird.

Stufe B

1,47 g 4-[3,3-Dimethoxy-5α,17β-dihydroxy-17α-(ethylthiomethyl)-estr-9-en-11β-yl] -benzaldehyd-1-ethylenketal wird in 15 ml Aceton mit 140 mg p-Toluolsulfonsäure innerhalb von 4 h bei Raumtemperatur umgesetzt. Man gießt in wäßrige Bicarbonat-Lösung ein, saugt ab und wäscht neutral. Der Niederschlag (1,24 g) wird durch Chromatographie gereinigt. Man erhält 640 mg
4-[17α-Ethylthiomethyl-17β-hydroxy-3-oxoestra-4,9-dien-11β-yl]benzaldehyd.
Schmp.: 180 - 182°C (Aceton);
$\alpha_D$ = + 160° ($CHCl_3$) ;
IR in KBr [$cm^{-1}$] : 1650, 1656 (C=C-C=C-C=O), 1697 (CH=O); UV [MeOH] : $\lambda_{max}$ 264 nm; ε = 20 375, log ε = 4.31, $\lambda_{max}$ = 299 nm, ε = 22 810, log ε = 4.36.
$^1$H-NMR [$CDCl_3$; TMS] :0,54 (s, 3H, H-18) ; 1,29 (t, 3H, $SCH_2CH_3$) ; 2,61 (m, 2H, $SCH_2CH_3$) ; 2,88 (s, 1H, OH); 2,71 und 2,95 (2d, 2H, J = 12,9 Hz; $CH_2S$) ; 4,48 (d, 1H, J = 7,2 Hz, H-11α); 5,80 (s, 1H, H-4); 7,37 (d, 2H, J = 8,1 Hz, H-2') ; 7,81 (d, 2 H, J = 8,4 Hz, H-3') ; 9,98 (s, 1H, CH=O).

Stufe C

392 mg 4-[17β-Hydroxy-17α-ethylthiomethyl-3-oxoestra-4,9-dien-11β-yl]benzaldehyd wird in 20 ml Pyridin mit 61 mg Hydroxylaminhydrochlorid bei Raumtemperatur innerhalb von 3,5 h umgesetzt. Man gießt in Eiswasser ein und saugt den farblosen Niederschlag ab, wäscht mit Wasser neutral und trocknet unter Vakuum. 560 mg Rohprodukt werden durch Chromatographie gereinigt und aus Aceton umkristallisiert. Man erhält 335 mg 4[17β-Hydroxy-17α-ethylthiomethyl-3-oxoestra-4,9-dien-11β-yl]benzaldehyd-(E)oxim.
Schmp.: 132 - 137 °C (Aceton); $\alpha_D$ = + 165 ° ($CHCl_3$) ;
IR in KBr [$cm^{-1}$] : 1649, 1655 (C=C-C=C-C=O);
UV [MeOH] : $\lambda_{max}$ 264 nm ε = 23 800, log ε= 4.38, $\lambda_{max}$ 299 nm, ε = 23 045, log ε = 4.36;
$^1$H-NMR: [$CDCl_3$; TMS] : 0,56 (s, 3H, H-18); 1,29 (t 3H, J = 7,5 Hz, $SCH_2CH_3$) ; 2,61 (m, 2H; $SCH_2CH_3$) ; 2,71 und 2,96 (2d, 2H, J = 12,9 Hz, 17α-$CH_2SC_2CH_3$) ; 2,90 (s, 1H, OH) ; 4,42 (d, 1H, J = 7,2 Hz, H-11α) ; 5,79 (s, 1H, H-4); 7,20 (d, 2H, J = 8,4 Hz, H-2'); 7,49 (d, 2H, J = 8,4 Hz, H-3'); 7,93 (s, 1H, NOH) ; 8,10 (s, 1H CH=N).

Beispiel 10

(17R)-4-{3-Oxoestra-4,9-dien-17-spiro-5'-oxazolidin-2'on-11β-yl}-benzaldehyd-1-(E)-[O-(ethylthio)carbonyl]-oxim

Schmp.: 133 -138 und 150-158 °C (Aceton/Ethanol);
$\alpha_D$ = + 193 ° ($CHCl_3$) ;
IR in KBr [$cm^{-1}$] : 1658 (C=C-C=C-C=O), 1719 (C=O) ;
UV [MeOH] : $\lambda_{max}$ 273 nm ε = 26 830, log ε= 4.43, $\lambda_{max}$ 298 nm ε = 24 240, log ε = 4.38;
$^1$H-NMR: [$CDCl_3$; TMS] : 0,53 (s, 3H, H-18) ; 1,37 (t, 3H, J = 7,5 Hz, $SCH_2CH_3$) ; 2,95 (q, 2H; $SCH_2CH_3$) ; 3,81(d, 1H, J = 11,7 Hz, 17α-$CH_2S$-) ; 4,43 (d, 1H, J = 6,6 Hz, H-11α) ; 4,52 (d, 1H, J = 11,7 Hz, 17α-$CH_2S$-) ; 4,54 (s, 1H, NH) ; 5,80 (s, 1H, H-4) ; 7,28 (d, 2H, J = 8,4 Hz, H-2') ; 7,63 (d, 4H, J = 8,4 Hz, H-3') ; 8,32 (s, 1H CH=N).

Herstellung der Ausgangsverbindung

Stufe A

1,86 g 4-[17α-Chlormethyl-17β-hydroxy-3-oxoestra-4,9-dien-11β-yl]-benzaldehyd werden in 50 ml $CH_2Cl_2$ mit 0,76 ml Trichloracetylisocyanat innerhalb von einer Stunde bei Raumtemperatur umgesetzt. Nach Zugabe von wäßriger $NH_4Cl$-Lösung werden die Phasen getrennt. Die organische Phase wird neutral gewaschen, getrocknet und unter

Vakuum eingedampft. Man erhält 4-[17α-Chlormethyl-17β-(trichloracetylcarbamoyl)-3-oxoestra-4,9-dien-11β-yl]-benzaldehyd als Schaum, der in Methylenchlorid aufgenommen wird. Die Lösung wird zusammen mit 10 g Aluminiumoxid (Woelm super I basisch) 5 h bei Raumtemperatur gerührt. Danach wird abfiltriert, mit Methylenchlorid und Methanol nachextrahiert. Das Lösungsmittel wird verdampft und der braune Schaum durch Chromatographie an Kieselgel gereinigt. Es werden 850 mg (17R)-4-(3-Oxoestra-4,9-dien-17-spiro-5'oxazolidin-2'-on-11β-yl)-benzaldehyd isoliert.
Schmp.: 173 °C Zersetzung (Methyl-tert.butylether / Aceton/Hexan) ;
$\alpha_D$ = + 152 ° ($CHCl_3$) ;
IR in KBr [$cm^{-1}$] : 1603 (Phenyl), 1650, 1682 (C=C-C=C-C=O), 1701,1721 (C=O) ;
UV [MeOH] : $\lambda_{max}$ 262 nm, ε = 18 090, log ε = 4.26, $\lambda_{max}$ 297 nm, ε = 20 760, log ε = 4.32;
$^1$H-NMR: [$CDCl_3$; TMS] : 0,53 (s, 3H, H-18) ; 3,82(d, 1H, J = 11,7 Hz, 17α-$CH_2$NH-) ; 4,48 (d, 1H, J = 6,9 Hz, H-11α); 4,52(d, 1H, J = 11,7 Hz, 17α-$CH_2$NH-) ; 4,59 (s,1H, NH) ; 5,81 (s, 1H, H-4); 7,39 (d, 2H, J = 8,4 Hz, H-2') ; 7,82 (d, 4H, J = 8,4 Hz, H-3') ; 9,98 (s, 1H CH=O), nach TAI-Zugabe: 0,56 (s, 3H, H-18); 3,90 (d, 1H, J = 12,3 Hz, 17α-$CH_2$NH-) ; 4,37 (d, 1H, J = 12,3 Hz, 17α-$CH_2$NH-); 4,52 (d, 1H, J = 7,2 Hz, H-11α); 5,83 (s, 1H, H-4) ; 7,38 (d, 2H, J = 8,1 Hz, H-2') ; 7,82 (d, 4H, J = 8,1 Hz, H-3') ; 9,98 (s, 1H CH=O), 10,45 (breites s, 1 H, NCONHC=O$CCl_3$).

Stufe B

440 mg (17R)-4-(3-Oxoestra-4,9-dien-17-spiro-5'-oxazolidin-2'-on-11β-yl)-benzaldehyd werden in 10 ml Pyridin mit 70 mg Hydroxylaminhydrochlorid innerhalb von 4 h bei Raumtemperatur umgesetzt. Man gießt in Eiswasser ein, saugt den Niederschlag ab, trocknet und reinigt durch Chromatographie. Nach Umkristallisation aus Aceton/ methyltert.butylether werden 180 mg (17R)-4-(3-Oxoestra-4,9-dien-17-spiro-5'-oxazolidin-2'-on-11β-yl)-benzaldehyd-1-(E)-oxim erhalten.
Schmp. : 181 °C Zersetzung (Methyl-tert.butylether / Aceton);
$\alpha_D$ = + 183 ° (DMSO) ;
IR in KBr [$cm^{-1}$] : 1614 (Phe-nyl), 1660, 1694 (C=C-C=C-C=O), 1729 (C=O);
UV [MeOH] : $\lambda_{max}$ 264 nm ε = 21 930, log ε= 4.34, $\lambda_{max}$ 298 nm ε = 21 930, log ε = 4.34;
$^1$H-NMR: [DMSO; TMS]: 0,47 (s, 3H, H-18); 3,97 (d, 1H, J = 10,8 Hz, 17α-$CH_2$NH-) ; 4,46 (d, 1H, J = 6,3 Hz, H-11α) ; 4,58 (d, 1H, J = 11,4 Hz, 17α-$CH_2$NH-) ; 4,59 (s,1H, NH) ; 5,69 (s, 1H, H-4); 6,3 und 6,5 (breite s, NH); 7,21 (d, 2H, J = 8,1 Hz, H-2') ; 7,51 (d, 4H, J = 8,4 Hz, H-3') ; 8,08 (s, 1H CH=N), 11,17 (s, 1H, OH).

Beispiel 11

Messung der Rezeptor-Bindungsaffinität

Die Rezeptor-Bindungsaffinität wurde bestimmt durch kompetitive Bindung eines spezifisch bindenden $^3$H-markierten Hormons (Tracer) und der zu testenden Verbindung an Rezeptoren im Cytosol aus tierischen Target-Organen. Dabei wurden Rezeptorsättigung und Reaktionsgleichgewicht angestarebt. Folgende Inkubationsbedingungen wurden gewählt:

Progesteron-Rezeptor:

Uterus-Cytosol des Estradiol-geprimten Kaninchens, aufbewahrt bei -30° C.
Puffer für Homogenisation und Inkubation: TED-Puffer 20 mM Tris/HCl, $p_H$ = 7,4; 1mM Ethylendiamintetraacetat, 2 mM Dithiothreitol) mit 250mM Saccharose.
Tracer: $^3$H-ORG 2058, 5nM;

Referenzsubstanz: Progesteron.

Glucocorticoid-Rezeptor:

Thymus-Cytosol der adrenalectomierten Ratte. Thymi aufbewahrt bei -30 °C.
Puffer: TED.
Tracer: $^3$H-Dexamethason, 20 nM.
Referenzsubstanz: Dexamethason.
Nach einer Inkubation von Rezeptorfraktion, Tracer und Kompetitor für 18 h bei 0-4°C erfolgte die Trennung von gebundenem und freiem Steroid durch Einmischen von Aktivkohle/Dextran (1% / 0,1%), Abzentrifugieren und Messung der rezeptorgebundenen $^3$H-Aktivität im Überstand.

Aus der Messung in Konzentrationsreihen wurden die $IC_{50}$ für die Referenzsubstanz und für die zu testende Verbindung ermittelt und als Quotient beider Werte (x 100 %) die relative molare Bindungsaffinität bestimmt.

Beispiel 12

Hemmung der frühen Gravidität bei der Ratte

Rattenweibchen im Gewicht von 180-200 g wurden im Stadium Prooestrus angepaart. Bei Nachweis von Spermien im Vaginalbereich am nächsten Tag wird dieser als Tag 1 (d =1) der Gravidität gesetzt. Die Behandlung der Ratten mit Testsubstanz oder Vehikel erfolgt mit 0,2 ml Benzoylbenzoat / Rizinusöl (1+4 v/v) subcutan vom Tag 5 bis Tag 7 (d5 -d7), die Autopsie erfolgt am Tag d 9 schmerzlos. Die Uterushörner wurde präpariert und auf intakte oder geschädigte Nidationstellen hin untersucht. Die Rate von vollständig gehemmten Graviditäten in den einzelnen Gruppen ergibt sich aus Tabelle 2.

Beispiel 13

Hemmung der späten Gravidität bei graviden Meerschweinchen

Gravide Meerschweinchen werden von Tag 43-44 mit Testsubstanzen behandelt. Versuchstieren wurde die Testsubstanz in oeliger Lösung (0,2-2,0ml Benzylbenzoat/Rizinusoel 1+4 v/v) 1x täglich am Tag 43 und 44 subkutan injiziert. Kontrolltiere wurden mit Vehikel behandelt. Die Gravidität der Tiere wurde bis Tag 50 verfolgt, insbesondere wurde auf die Ausstoßung von Foeten und Placenten beobachtet und registriert.

Beispiel 14

Antiluteolysetest/Ovulationshemmtest am zyklischen Meerschweinchen:

Dieser Test basiert darauf, daß Progesteron zum Zyklusende die uterine Prostaglandinsekretion stimuliert. Die Hemmung dieser Funktion führt beim Meerschweinchen zu einer Persistenz der Corpora lutea (Antiluteolyse).

Progesteronrezeptorantagonisten mit partialagonistischer Aktivität sind in diesem Test gering (siehe RU 486) oder gar nicht antiluteolytisch wirksam. Progesteron-agonistische Aktivität kann ebenfalls in diesem Versuchsdesign nachgewiesen werden, zum einen durch antiovulatorische Aktivität, zum anderen aber durch Nachweis typischer Progesteroneffekte im Genitaltrakt. Dieser Test erlaubt also die therapierelevante Typisierung von Progesteronantagonisten als "reine" oder "agonistische" Substanzen.

Meerschweinchen werden am Tag 10 bis Tag 17 des Zyklus mit der Testsubstanz behandelt. Am Tag 10 vor der Substanzbehandlung sowie an den darauffolgenden Tagen bis zur Autopsie, werden die Progesteronkonzentrationen im Serum bestimmt (Elger, W., Neef, G., Beier, S., Fähnrich, M., Grundel, M. et al. in Current Concepts in Fertility Regulation and Reproduction, (eds). Puri, C.P. and Van Look, P.F.H. (1994) 303-328).

Beispiel 15

McPhail-Test an immaturen weiblichen Kaninchen:

Das Endometrium sexuell unreifer Kaninchen reagiert auf Gestagene mit einer typischen histologischen Umwandlung. Diese ist die Grundlage des McPhail-Testes. Dieser wurde eingesetzt um zu prüfen, ob Substanzen, die Gegenstand dieser Erfindung sind, progesteronähnliche Eigenschaften besitzen. Daneben wurde bei simultaner Behandlung mit maximal transformierender Dosis von Progesteron die antagonistische Partialwirkung dieser Substanzen untersucht. Die Wirkung im McPhail-Test wird durch die "McPhail Scores" 1-4 erfaßt, wobei Score 4 einer maximalen Transformation entspricht.

**Patentansprüche**

1. S-substituierte 11β-Benzaldoxim-estra-4,9-dienkohlensäurethiolester der allgemeinen Formel I,

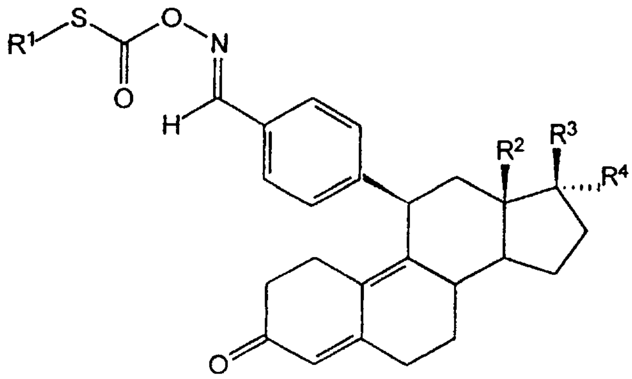

(I)

worin

$R^1$ ein Alkylrest mit 1-10 Kohlenstoffatomen, ein Arylrest mit 6-10 Kohlenstoffatomen oder ein Alkylaryl- oder Arylalkylrest mit jeweils 7-10 Kohlenstoffatomen ist,

$R^2$ für einen Alkylrest mit 1-3 Kohlenstoffatomen oder ein Wasserstoffatom steht,

$R^3$ eine Hydroxylgruppe, eine O-Alkylgruppe mit 1-10 Kohlenstoffatomen, eine O-Arylgruppe mit 6-10 Kohlenstoffatomen, eine O-Aralkyl-, oder O-Alkylarylgruppe mit jeweils 7-10 Kohlenstoffato-men, einen Rest - $OCOR^5$, $-OCONHR^5$ oder $-OCOOR^5$ bedeutet,

wobei

$R^5$ ein Wasserstoffatom, eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen bedeutet,

$R^4$ für ein Wasserstoffatom, eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen steht,

einen Rest $-(CH_2)_nCH_2Y$ bedeutet,

wobei

$n = 0$, 1 oder 2 ist,

Y für ein Fluor-, Chlor-, Brom- oder Iodatom, für eine Cyano-, Amino-, Azido- oder Rhodanogruppe steht, oder einen Rest $-OR^6$, $-SR^6$, $-(CO)SR^6$ oder - $(CO)OR^6$ bedeutet, wobei

$R^6$ ein Wasserstoffatom, eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen bedeutet,

oder ein Rest $-COR^5$ ist, wobei $R^5$ die oben angegebene Bedeutung hat,

einen Rest $-OR^5$ oder $-OCOR^5$ darstellt, wobei $R^5$ die oben angegebene Bedeutung hat,

einen Rest $-(CH_2)_m-CH=CH(CH_2)_p-R^6$ bedeutet,

wobei

$m = 0$, 1, 2 oder 3 ist,

$p = 0$, 1 oder 2 bedeutet und

$R^6$ die oben angegebene Bedeutung hat oder einen Rest $-OR^5$ oder $-OCOR^5$ darstellt, wobei $R^5$ die oben angegebene Bedeutung hat,

einen Rest $-(CH_2)_oC\equiv CR^7$ darstellt,

wobei

$o = 0$, 1 oder 2 ist und

$R^7$ für ein Wasserstoffatom, ein Fluor-, Chlor-,

Brom- oder Iodatom, für eine Alkylgruppe mit 1-10 Kohlenstoffatomen, eine Arylgruppe mit 6-10 Kohlenstoffatomen, einen Aralkyl- oder Alkylarylrest mit jeweils 7-10 Kohlenstoffatomen steht oder einen Rest $-OR^5$ darstellt, wobei $R^5$ die oben angegebene Bedeutung hat,

oder einen Rest $-OCOR^5$ bedeutet, wobei $R^5$ die oben angegebene Bedeutung hat,

oder einen Rest $-CH_2OR^5$ darstellt, wobei $R^5$ die

oben angegebene Bedeutung hat,

oder einen Rest $-C\equiv CCH_2OH$ bedeutet,

oder $R^3$ und $R^4$ zusammen einen beliebig substituierten fünf- oder sechsgliedrigen Ring mit mindestens einem Kohlenstoffatom und 0 - 4 Heteroatomen aus der Gruppe Sauerstoff, Schwefel, Selen, Tellur, Stickstoff, Phosphor, Silicium oder Germanium bilden

und deren pharmazeutisch annehmbare Salze.

**2.** Verbindungen nach Anspruch 1, **dadurch gekennzeichnet, daß** $R^1$ ein Alkylrest mit 1-6 Kohlenstoffatomen ist.

**3.** Verbindungen nach mindestens einem der vorhergehenden Ansprüche, **dadurch gekennzeichnet, daß** $R^2$ eine Methyl- oder Ethylgruppe ist.

**4.** Verbindungen nach mindestens einem der vorhergehenden Ansprüche, **dadurch gekennzeichnet, daß** $R^3$ eine Hydroxylgruppe darstellt.

**5.** Verbindungen nach mindestens einem der Ansprüche 1-3, **dadurch gekennzeichnet, daß** $R^3$ eine O-Alkylgruppe mit 1-6 Kohlenstoffatomen darstellt.

**6.** Verbindungen nach mindestens einem der vorhergehenden Ansprüche, **dadurch gekennzeichnet, daß**
$R^4$ für einen Rest $-OR^5$ oder $-OCOR^5$ steht, wobei
$R^5$ einen Alkylrest mit 1-6 Kohlenstoffatomen bedeutet.

**7.** Verbindungen nach mindestens einem der Ansprüche 1-5, **dadurch gekennzeichnet, daß**
$R^4$ für einen Rest $-(CH_2)_m-CH=CH(CH_2)_p-R^6$ steht, wobei
m = 1 und p = 1 ist und
$R^6$ für einen Alkylrest mit 1-6 Kohlenstoffatomen
oder eine Gruppe $-OR^5$ oder $-OCOR^5$ steht, wobei
$R^5$ ein Wasserstoffatom oder eine Alkylgruppe mit 1-6 Kohlenstoffatomen darstellt.

**8.** Verbindungen nach mindestens einem der Ansprüche 1-5, **dadurch gekennzeichnet, daß**
$R^4$ für einen Rest $-(CH_2)_m-CH=CH(CH_2)_p-R^6$ steht, wobei
m = 0 und p = 1 ist und
$R^6$ für eine Gruppe $-OR^5$ oder $-OCOR^5$ steht, wobei
$R^5$ ein Wasserstoffatom oder eine Alkylgruppe mit 1-6 Kohlenstoffatomen ist.

**9.** Verbindungen nach mindestens einem der Ansprüche 1-5, **dadurch gekennzeichnet, daß**
$R^4$ für einen Rest $-(CH_2)_oC\equiv CR^7$ steht, wobei
o = 1 ist und
$R^7$ für eine Alkylgruppe mit 1-6 Kohlenstoffatomen steht oder einen Rest $-OCOR^5$ bedeutet, oder für einen Rest $-CH_2OR^5$ steht, wobei
$R^5$ einen Alkylrest mit 1-6 Kohlenstoffatomen oder ein Wasserstoffatom darstellt.

**10.** Verbindungen nach mindestens einem der Ansprüche 1-5, **dadurch gekennzeichnet, daß**
$R^4$ für einen Rest-$(CH_2)_nCH_2Y$ steht, wobei
n = 0 oder 1 ist,
Y für ein F-, Cl-, Br- oder Iodatom, für eine Cyano-, Amino-, Azido- oder Rhodanogruppe steht, oder ein Rest $-OR^6$ oder $-SR^6$, $-(CO)SR^6$ oder $-(CO)OR^6$ ist, wobei
$R^6$ ein Wasserstoffatom oder eine Alkylgruppe mit 1-10 Kohlenstoffatomen darstellt.

**11.** Verbindungen nach mindestens einem der Ansprüche 1-3, **dadurch gekennzeichnet, daß**
$R^3$ und $R^4$ zusammen einen beliebig substituierten fünf- oder sechsgliedrigen Ring mit mindestens einem Kohlenstoffatom und 0 - 4 Heteroatomen bilden, wobei
die Heteroatome aus der Gruppe Sauerstoff, Schwefel und Stickstoff stammen.

**12.** Verbindungen nach Anspruch 11, **dadurch gekennzeichnet, daß** ein fünfgliedriger Ring ausgebildet wird, der 1 oder 2 Heteroatome enthält.

**13.** Verbindungen nach Anspruch 12, **dadurch gekennzeichnet, daß** der Ring einen Heterocyclus aus der Gruppe Oxazolidinon, Oxazolinon, Thiazolidinon, Thiazolinon, Imidazolidinon, Imidazolinon, 1,3-Dioxolanon, 1,3-Dioxolenon, 1,3-Oxathiolanon, 1,3-Oxathiolenon, Pyrrolidinon, Pyrrolinon, Oxazolidinthion, Oxazolinthion, Thiazolidinthion, Thiazolinthion, Imidazolidinthion, Imidazolinthion, Dioxolanthion, Pyrrolidinthion und Pyrrolinthion darstellt.

**14.** Verbindungen nach Anspruch 13, **dadurch gekennzeichnet, daß** der fünfgliedrige Ring ein Oxazolidin-2-on. oder ein Oxazolidin-2-thion ist.

**15.** Verbindungen nach Anspruch 1, nämlich
4-[17β-Methoxy-17α-(methoxymethyl)-3-oxoestra-4, 9-dien-11β-yl]-benzaldehyd-1-(E)-[O-(methylthio)carbonyl]oxim,
4-[17β-Hydroxy-17α-(methoxymethyl)-3-oxoestra-4,9-dien-11β-yl]-benzaldehyd-1-(E)-[O-(methylthio)carbonyl]oxim,
4-[17β-Methoxy-17α-(methoxymethyl)-3-oxoestra-4,9-dien-11β-yl]-benzaldehyd-1-(E)-[O-(ethylthio)carbonyl]oxim,
4- [17ß-Methoxy-17α-(methoxymethyl)-3-oxoestra-4,9-dien-11β-yl]-benzaldehyd-1-(Z)-[O-(ethylthio)carbonyl]oxim,
4-[17β-Methoxy-17α-(ethoxymethyl)-3-oxoestra-4,9-dien-11β-yl]-benzaldehyd-1-(E)-[O-(ethylthio)carbonyl]oxim,
4-[17β-Hydroxy-17α-(methoxymethyl)-3-oxoestra-4,9-dien-11β-yl]-benzaldehyd-1-(E)-[O-(n-propylthio)-carbonyl)oxim,
4-[17β-Methoxy-17α-(n-propoxymethyl)-3-oxoestra-4,9-dien-11β-yl]-benzaldehyd-1-(E)-[O-(methylthio)-carbonyl]oxim,
4-[17β-Hydroxy-17α-(i-propoxymethyl)-3-oxoestra-4,9-dien-11β-yl]-benzaldehyd-1-(E)-[O-(ethylthio)-carbonyl]oxim,
4-[17β-Hydroxy-17α-(methoxymethyl)-3-oxoestra-4,9-dien-11β-yl]-benzaldehyd-1-(E)-[O-(ethylthio)-carbonyl]oxim,
4-[17β-Hydroxy-17α-Z-(3-hydroxypropenyl)-3-oxo-estra-4,9-dien-11β-yl]-benzaldehyd-1-(E)-[O-(methylthio)carbonyl]oxim,
4-[17β-Hydroxy-17α-E-(3-hydroxypropenyl)-3-oxo-estra-4,9-dien-11β-yl]-benzaldehyd-1-(E)-[O-(methyl-thio)-carbonyl]oxim,
4-[17β-Methoxy-17α-(3-hydroxy-1-propinyl)-3-oxoestra-4,9-dien-11β-yl]-benzaldehyd-1-(E)-[O-(ethylthio)carbonyl]oxim.
4-[17β-Hydroxy-17α-(azidomethyl)-3-oxoestra-4,9-dien-11β-yl]-benzaldehyd-1-(E)-[O-(ethylthio)-carbonyl]-oxim,
4-[17β-Hydroxy-17α-(chlormethyl)-3-oxoestra-4,9-dien-11β-yl]-benzaldehyd-1-(E)-[O-(ethylthio)carbonyl]-oxim,
4-[17β-Ethoxy-17α-(chlormethyl)-3-oxoestra-4,9-dien-11β-yl]-benzaldehyd-1-(E)-[O-(methylthio)-carbonyl]oxim,
4-[17β-Hydroxy-17α-(cyanomethyl)-3-oxoestra-4,9-dien-11β-yl]-benzaldehyd-1-(E)-[O-(ethylthio)carbonyl]-oxim,
4-[17β-Hydroxy-17α-(ethylthiomethyl)-3-oxoestra-4,9-dien-11β-yl]-benzaldehyd-1-(E)-[O-(ethylthio)-carbonyl]oxim,
4-[17β-Hydroxy-17α-(ethylthiomethyl)-3-oxoestra-4,9-dien-11β-yl]-benzaldehyd-1-(E)-[O-(methylthio)-carbonyl]oxim,
4-[17β-Ethoxy-17α-(methylthiomethyl)-3-oxoestra-4,9-dien-11β-yl]-benzaldehyd-1-(E)-[O-(ethylthio)-carbonyl]oxim,
4-[17β-Hydroxy-17α-[(ethylthiocarbonyl]methyl)-3-oxoestra-4,9-dien-11β-yl]-benz-aldehyd-1-(E)-[O-(ethylthio)carbonyl)oxim,
4-[17β-Hydroxy-17α-(aminomethyl)-3-oxoestra-4,9-dien-11β-yl]-benzaldehyd-1-(E)-[O-(ethylthio)carbonyl]-oxim und
(17R)-4-{3-Oxoestra-4,9-dien-17-spiro-5'-oxazolidin-2'-on-11β-yl}-benzaldehyd-1-(E)-[O-(ethylthio)-carbonyl]oxim.

**16.** Verfahren zur Herstellung von S-substituierten 11β-Benzaldoxim-estra-4,9-dien-kohlensäurethiolester der allgemeinen Formel I,

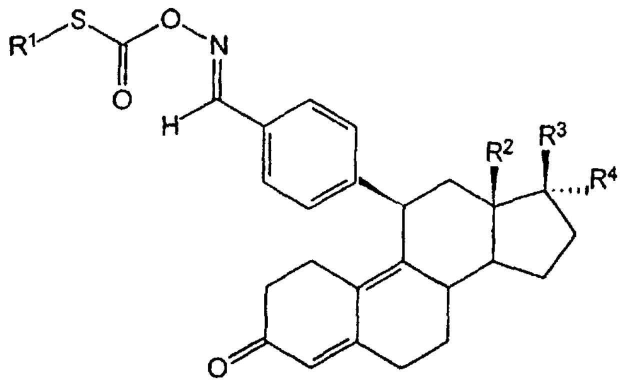

(I)

worin $R^2$, $R^3$ und $R^4$ die im Anspruch 1 angegebene Bedeutung haben, **dadurch gekennzeichnet, daß** man in an sich bekannter Weise eine Verbindung der allgemeinen Formel II,

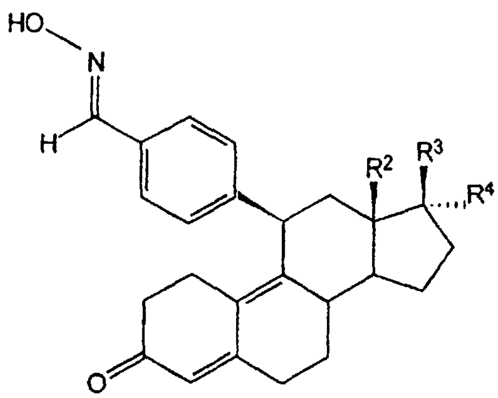

(II)

worin $R^2$, $R^3$ und $R^4$ dieselbe Bedeutung haben wie $R^2$, $R^3$ und $R^4$ in der Formel I, durch Behandlung mit einem Ameisensäurederivat der Formel III

$$Nuc\text{-}(CO)\text{-}SR^1 \qquad (III)$$

worin

$R^1$ die in Anspruch 1 genannte Bedeutung hat und Nuc für ein Nucleophil steht, in einem Lösemittel umsetzt und in eine Verbindung der allgemeinen Formel I überführt.

**17.** Verfahren gemäß Anspruch 16, **dadurch gekennzeichnet, daß** man als Lösemittel ein tertiäres Amin einsetzt und die Umsetzung bei einer Temperatur zwischen 20 °C und 80 °C durchführt.

**18.** Verfahren nach Anspruch 17, **dadurch gekennzeichnet, daß** man die Umsetzung mit Chlorameisensäurethioestern in Pyridin oder Triethylamin bei einer Temperatur zwischen 20 °C und 40 °C durchführt.

**19.** Pharmazeutische Zusammensetzungen, **gekennzeichnet durch** den Gehalt an mindestens einer Verbindung nach einem der Ansprüche 1 bis 15.

**20.** Verwendung von Verbindungen nach einem der Ansprüche 1 bis 15 zur Herstellung eines Arzneimittels zur Behandlung von Endometriose, Uterus myomatosus, Dysmennorrhoe und praemenstruellem Syndrom, zur Induktion einer reversiblen Amennorhoe ohne Oestrogendefizit und für die klimakterische Substitutionstherapie (Hormone Replacement Therapy (HRT)) gegebenenfalls in Kombination mit Oestrogenen.

**21.** Verwendung von Verbindungen nach einem der Ansprüche 1 bis 15 zur Herstellung von Kontrazeptiva.

## Claims

**1.** S-substituted 11β-benzaldoxime estra-4,9-diene-carbonic acid thioesters of the general formula I,

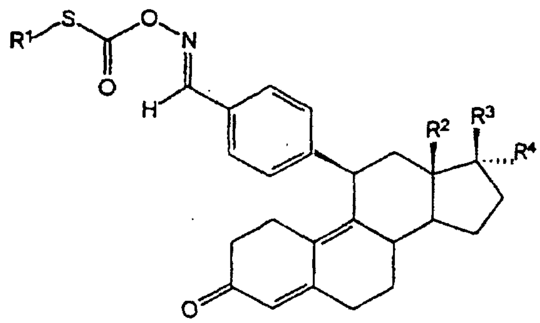

(I)

in which

$R^1$ is an alkyl radical having 1-10 carbon atoms, an aryl radical having 6-10 carbon atoms or an alkylaryl or arylalkyl radical each having 7-10 carbon atoms,

$R^2$ is an alkyl radical having 1-3 carbon atoms or a hydrogen atom,

$R^3$ is a hydroxyl group, an O-alkyl group having 1-10 carbon atoms, an O-aryl group having 6-10 carbon atoms, an O-aralkyl, or O-alkylaryl group each having 7-10 carbon atoms, a radical $-OCOR^5$, $-OCONHR^5$ or $-OCOOR^5$, where

$R^5$ is a hydrogen atom, an alkyl group having 1-10 carbon atoms, an aryl group having 6-10 carbon atoms, an aralkyl or alkylaryl group each having 7-10 carbon atoms;

$R^4$ is a hydrogen atom, an alkyl group having 1-10 carbon atoms, an aryl group having 6-10 carbon atoms, an aralkyl or alkylaryl radical each having 7-10 carbon atoms,

a radical $-(CH_2)_nCH_2Y$,

where

n = 0, 1 or 2,

Y is a fluorine, chlorine, bromine or iodine atom, a cyano, amino, azido or thiocyano group, or is a radical $-OR^6$, $-SR^6$, $-(CO)SR^6$ or $-(CO)OR^6$,

where

$R^6$ is a hydrogen atom, an alkyl group having 1-10 carbon atoms, an aryl group having 6-10 carbon atoms, an aralkyl or alkylaryl radical each having 7-10 carbon atoms,

or is a radical $-COR^5$, where $R^5$ has the meaning indicated above,

is a radical $-OR^5$ or $-OCOR^5$, where $R^5$ has the meaning indicated above,

is a radical $-(CH_2)_m-CH=CH(CH_2)_p-R^6$,

where

m = 0, 1, 2 or 3,

p = 0, 1 or 2 and

$R^6$ has the meaning indicated above or is a radical $-OR^5$ or $-OCOR^5$, where $R^5$ has the meaning indicated above,

is a radical $-(CH_2)_oC\equiv CR^7$,

where

o = 0, 1 or 2 and

$R^7$ is a hydrogen atom, a fluorine, chlorine, bromine or iodine atom, an alkyl group having 1-10 carbon atoms, an aryl group having 6-10 carbon atoms, an aralkyl or alkylaryl radical each having 7-10 carbon atoms or is a

radical $-OR^5$, where $R^5$ has the meaning indicated above,
or is a radical $-OCOR^5$, where $R^5$ has the meaning indicated above,
or is a radical $-CH_2OR^5$, where $R^5$ has the meaning indicated above,
or is a radical $-C{\equiv}CCH_2OH$,
or $R^3$ and $R^4$ together form an arbitrarily substituted five- or six-membered ring having at least one carbon atom and 0-4 heteroatoms from the group consisting of oxygen, sulfur, selenium, tellurium, nitrogen, phosphorus, silicon or germanium,
and their pharmaceutically acceptable salts.

**2.** Compounds according to Claim 1, **characterized in that** $R^1$ is an alkyl radical having 1-6 carbon atoms.

**3.** Compounds according to at least one of the preceding claims, **characterized in that** $R^2$ is a methyl or ethyl group.

**4.** Compounds according to at least one of the preceding claims, **characterized in that** $R^3$ is a hydroxyl group.

**5.** Compounds according to at least one of Claims 1-3, **characterized in that** $R^3$ is an O-alkyl group having 1-6 carbon atoms.

**6.** Compounds according to at least one of the preceding claims, **characterized in that**
$R^4$ is a radical $-OR^5$ or $-OCOR^5$, where
$R^5$ is an alkyl radical having 1-6 carbon atoms.

**7.** Compounds according to at least one of Claims 1-5, **characterized in that**

$$R^4 \text{ is a radical } -(CH_2)_m-CH{=}CH(CH_2)_p-R^6\text{, where}$$

m = 1 and p = 1 and
$R^6$ is an alkyl radical having 1-6 carbon atoms or a group $-OR^5$ or $-OCOR^5$, where
$R^5$ is a hydrogen atom or an alkyl group having 1-6 carbon atoms.

**8.** Compounds according to at least one of Claims 1-5, **characterized in that**
$R^4$ is a radical $-(CH_2)_m-CH{=}CH(CH_2)_p-R^6$, where
m = 1 and p = 1 and
$R^6$ is a group $-OR^5$ or $-OCOR^5$, where
$R^5$ is a hydrogen atom or an alkyl group having 1-6 carbon atoms.

**9.** Compounds according to at least one of Claims 1-5, **characterized in that**
$R^4$ is a radical $-(CH_2)_oC{\equiv}CR^7$, where
o = 1 and
$R^7$ is an alkyl group having 1-6 carbon atoms or is a radical $-OCOR^5$, or
is a radical $-CH_2OR^5$, where
$R^5$ is an alkyl radical having 1-5 carbon atoms or a hydrogen atom.

**10.** Compounds according to at least one of Claims 1-5, **characterized in that**
$R^4$ is a radical $-(CH_2)_nCH_2Y$, where
n = 0 or 1,
Y is an F, Cl, Br or iodine atom, a cyano, amino, azido or thiocyano group,
or is a radical $-OR^6$ or -SR6, $-(CO)SR^6$ or $-(CO)OR^6$,
where
$R^6$ is a hydrogen atom or an alkyl group having 1-10 carbon atoms.

**11.** Compounds according to at least one of Claims 1-3, **characterized in that**
$R^3$ and $R^4$ together are an arbitrarily substituted five- or six-membered ring having at least one carbon atom and 0-4 heteroatoms, where
the heteroatoms originate from the group consisting of oxygen, sulfur and nitrogen.

**12.** Compounds according to Claim 11, **characterized in that** a five-membered ring is formed which contains 1 or 2

heteroatoms.

**13.** Compounds according to Claim 12, **characterized in that** the ring is a heterocycle from the group consisting of oxazolidinone, oxazolinone, thiazolidinone, thiazolinone, imidazolidinone, imidazolinone, 1,3-dioxolanone, 1,3-dioxolenone, 1,3-oxathiolanone, 1,3-oxathiolenone, pyrrolidinone, pyrrolinone, oxazolidinethione, oxazolinethione, thiazolidinethione, thiazolinethione, imidazolidinethione, imidazolinethione, dioxolane-thione, pyrrolidinethione and pyrrolinethione.

**14.** Compounds according to Claim 13, **characterized in that** the five-membered ring is an oxazolidin-2-one or an oxazolidine-2-thione.

**15.** Compounds according to Claim 1, namely
4-[17β-methoxy-17α-(methoxymethyl)-3-oxoestra-4,9-dien-11β-yl]benzaldehyde 1-(E)[O-(methylthio)carbonyl] oxime,
4-[17β-hydroxy-17α-(methoxymethyl)-3-oxoestra-4,9-dien-11β-yl]benzaldehyde 1-(E)[O-(methylthio)carbonyl]oxime,
4-[17β-methoxy-17α-(methoxymethyl)-3-oxoestra-4,9-dien-11β-yl]benzaldehyde 1-(E)[O-(ethylthio) carbonyl] oxime,
4-[17β-methoxy-17α-(methoxymethyl)-3-oxoestra-4,9-dien-11β-yl]benzaldehyde 1-(Z)[O-(ethylthio)carbonyl]oxime,
4-[17β-methoxy-17α-(ethoxymethyl)-3-oxoestra-4,9-dien-11β-yl]benzaldehyde 1-(E)[O-(ethylthio)carbonyl]oxime,
4-[17β-hydroxy-17α-(methoxymethyl)-3-oxoestra-4,9-dien-11β-yl]benzaldehyde 1(E) [O-(n-propylthio)carbonyl] oxime,
4- [17β-methoxy-17α-(n-propoxymethyl)-3-oxoestra-4,9-dien-11β-yl]benzaldehyde 1-(E)[O-(methylthio)carbonyl] oxime,
4-[17β-hydroxy-17α-(i-propoxymethyl)-3-oxoestra-4,9-dien-11β-yl]benzaldehyde 1-(E)[O-(ethylthio)carbonyl] oxime,
4-[17β-hydroxy-17α-(methoxymethyl)-3-oxoestra-4,9-dien-11β-yl]benzaldehyde 1-(E)[O-(ethylthio)carbonyl] oxime,
4- [17β-hydroxy-17α-Z-(3-hydroxypropenyl)-3-oxoestra-4,9-dien-11β-yl]benzaldehyde 1-(E)[O-(methylthio)carbonyl]oxime,
4-[17β-hydroxy-17α-E-(3-hydroxypropenyl)-3-oxoestra-4,9-dien-11β-yl]benzaldehyde 1-(E)[O-(methylthio)carbonyl]oxime,
4-[17β-methoxy-17α-(3-hydroxy-1-propinyl)-3-oxoestra-4,9-dien-11β-yl]benzaldehyde 1-(E)[O-(ethylthio)-carbonyl]oxime,
4-[17β-hydroxy-17α-(azidomethyl)-3-oxoestra-4,9-dien-11β-yl]benzaldehyde 1-(E)[O-(methylthio)carbonyl] oxime,
4-[17β-hydroxy-17α-(chloromethyl)-3-oxoestra-4,9-dien-11β-yl]benzaldehyde 1-(E)[O-(ethylthio)carbonyl]oxime,
4-[17β-ethoxy-17α-(chloromethyl)-3-oxoestra-4,9-dien-11β-yl] benzaldehyde 1-(E)[O-(methylthio)carbonyl] oxime,
4-[17β-hydroxy-17α-(cyanomethyl)-3-oxoestra-4,9-dien-11β-yl]benzaldehyde 1-(E)[O-(ethylthio)carbonyl]oxime,
4- [17β-hydroxy-17α-(ethylthiomethyl)-3-oxoestra-4,9-dien-11β-yl]benzaldehyde 1-(E)[O-(ethylthio)carbonyl]oxime,
4-[17β-hydroxy-17α-(ethylthiomethyl)-3-oxoestra-4,9-dien-11β-yl]benzaldehyde 1-(E)[O-(methylthio)carbonyl]oxime,
4-[17β-ethoxy-17α-(methylthiomethyl)-3-oxoestra-4,9-dien-11β-yl]benzaldehyde 1-(E)[O-(ethylthio)carbonyl]oxime,
4- [17β-hydroxy-17α-[(ethylthiocarbonyl]methyl)-3-oxoestra-4,9-dien-11β-yl]benzaldehyde 1-(E)[O-(ethylthio)carbonyl]oxime,
4-[17β-hydroxy-17α-(aminomethyl)-3-oxoestra-4,9-dien-11β-yl]benzaldehyde 1-(E)[O-(ethylthio)carbonyl]oxime and
(17R)-4-{3-oxoestra-4,9-diene-17-spiro-5'-oxazolidin-2'-on-11β-yl}benzaldehyde 1-(E)-[O-(ethylthio)carbonyl]oxime.

**16.** Process for the preparation of S-substituted 11β-benzaldoxime-estra-4,9-dienecarbonic acid thio-esters of the general formula I,

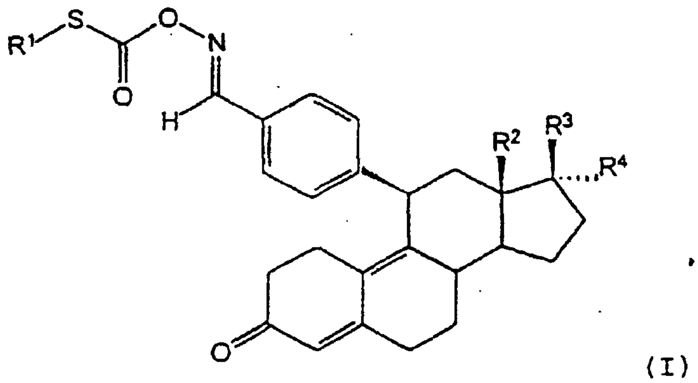

(I)

in which $R^2$, $R^3$ and $R^4$ have the meaning indicated in Claim 1, **characterized in that**, in a manner known per se, a compound of the general formula II,

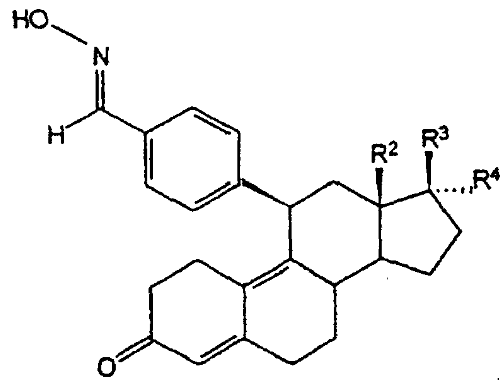

(II)

in which $R^2$, $R^3$ and $R^4$ have the same meaning as $R^{2'}$ $R^3$ and $R^4$ in the formula I, is reacted by treatment with a formic acid derivative of the formula III

$$Nuc\text{-}(CO)\text{-}SR^1 \quad (III)$$

in which
$R^1$ has the meaning mentioned in Claim 1 and
Nuc is a nucleophile, in a solvent and converted into a compound of the general formula I.

**17.** Process according to Claim 16, **characterized in that** the solvent employed is a tertiary amine and the reaction is carried out at a temperature between 20°C and 80°C.

**18.** Process according to Claim 17, **characterized in that** the reaction is carried out using chloroformic acid thioesters in pyridine or triethylamine at a temperature between 20°C and 40°C.

**19.** Pharmaceutical compositions, **characterized in that** they contain at least one compound according to one of Claims 1 to 15.

**20.** Use of compounds according to one of Claims 1 to 15 for the production of a medicament for the treatment of

endometriosis, uterus myomatosus, dysmenorrhea and premenstrual syndrome, for the induction of a reversible amenorrhea without estrogen deficit and for menopausal substitution therapy (Hormone Replacement Therapy HRT) if appropriate in combination with estrogens.

**21.** Use of compounds according to one of Claims 1 to 15 for the production of contraceptives.

## Revendications

**1.** Thiolesters S-substitués de 11β-benzaldoxime-estra-4,9-diène-acide carbonique de formule générale I :

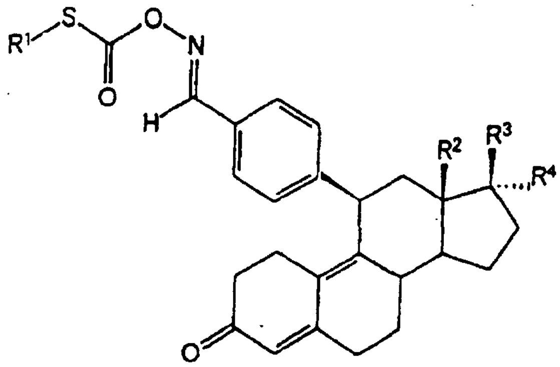

(I)

dans lesquelles
$R^1$ est un radical alkyle comportant de 1 à 10 atomes de carbone, un radical aryle comportant de 6 à 10 atomes de carbone ou un radical alkylaryle ou arylalkyle comportant respectivement de 7 à 10 atomes de carbone;
$R^2$ représente un radical alkyle comportant de 1 à 3 atomes de carbone ou un atome d'hydrogène,
$R^3$ est un groupe hydroxyle, un groupe O-alkyle comportant de 1 à 10 atomes de carbone, un groupe O-aryle comportant de 6 à 10 atomes de carbone, un groupe O-aralkyle ou O-alkylaryle comportant respectivement de 7 à 10 atomes de carbone, un radical $-OCOR^5$, $-OCONHR^5$, ou $-OCOOR^5$,
dans lequel
$R^5$ représente un atome d'hydrogène, un groupe alkyle comportant de 1 à 10 atomes de carbone, un groupe aryle comportant de 6 à 10 atomes de carbone ou un radical aralkyle ou alkylaryle comportant respectivement de 7 à 10 atomes de carbone.
$R^4$ représente un atome d'hydrogène, un groupe alkyle comportant de 1 à 10 atomes de carbone, un groupe aryle comportant de 6 à 10 atomes de carbone ou un radical aralkyle ou alkylaryle comportant respectivement de 7 à 10 atomes de carbone,
un radical $-(CH_2)_nCH_2Y$,
dans lequel
n = 0, 1 ou 2,
Y représente un atome de fluor, de chlore, de brome ou d'iode, un groupe cyano, amino, azido ou rhodano, ou un radical $-OR^6$, $-SR^6$,$-(CO)SR^6$ ou $-(CO)OR^6$, dans lesquels
$R^6$ représente un atome d'hydrogène, un groupe alkyle comportant de 1 à 10 atomes de carbone, un groupe aryle comportant de 6 à 10 atomes de carbone ou un radical aralkyle ou alkylaryle comportant respectivement de 7 à 10 atomes de carbone,
ou un radical $-COR^5$, dans lequel $R^5$ a la signification indiquée ci-dessus,
un radical $-OR^5$ ou $-OCOR^5$, dans lequel $R^5$ a la signification indiquée ci-dessus,
un radical $-(CH_2)_m-CH=CH(CH_2)_p-R^6$,
dans lequel
m = 0, 1, 2 ou 3
p = 0, 1 ou 2, et
$R^6$ a la signification indiquée ci-dessus ou représente un radical $-OR^5$ ou $-OCOR^5$, dans lesquels $R^5$ a la signification indiquée ci-dessus,
un radical $-(CH_2)_oC{\equiv}CR^7$,
dans lequel

o = 0, 1 ou 2,

$R^7$ représente un atome d'hydrogène, un atome de fluor, de chlore, de brome ou d'iode, un groupe alkyle comportant de 1 à 10 atomes de carbone, un groupe aryle comportant de 6 à 10 atomes de carbone, un radical aralkyle ou alkylaryle comportant respectivement de 7 à 10 atomes de carbone, ou représente un radical $-OR^5$, dans lequel $R^5$ a la signification indiquée ci-dessus,

ou représente un radical $-OCOR^5$, dans lequel $R^5$ a la signification indiquée ci-dessus,

ou représente un radical $-CH_2OR^5$, dans lequel $R^5$ a la signification indiquée ci-dessus,

ou un radical $-C{\equiv}CCH_2OH$,

ou $R^3$ et $R^4$ forment ensemble un cycle à cinq ou six chaînons substitué à n'importe quelle position comportant au moins un atome de carbone et de 0 à 4 hétéroatomes issus du groupe de oxygène, soufre, sélénium, tellure, azote, phosphore, silicium ou germanium,

ou leurs sels pharmaceutiquement acceptables.

**2.** Composés selon la revendication 1, **caractérisés en ce que** $R^1$ est un radical alkyle comportant de 1 à 6 atomes de carbone.

**3.** Composés selon au moins une des revendications précédentes, **caractérisés en ce que** $R^2$ est un groupe méthyle ou éthyle.

**4.** Composés selon au moins une des revendications précédentes, **caractérisés en ce que** $R^3$ représente un groupe hydroxyle.

**5.** Composés selon au moins une des revendications de 1 à 3, **caractérisés en ce que** $R^3$ représente un groupe O-alkyle comportant de 1 à 6 atomes de carbone.

**6.** Composés selon au moins une des revendications précédentes, **caractérisés en ce que** $R^4$ représente un radical $-OR^5$ ou $-OCOR^5$, dans lequel $R^5$ représente un radical alkyle comportant de 1 à 6 atomes de carbone.

**7.** Composés selon au moins une des revendications de 1 à 5, **caractérisés en ce que** $R^4$ représente un radical $-(CH_2)_m-CH=CH(CH_2)_p-R^6$, dans lequel
m = 1 et p = 1 et
$R^6$ représente un radical alkyle comportant de 1 à 6 atomes de carbone ou un groupe $-OR^5$ ou $-OCOR^5$, dans lequel
$R^5$ représente un atome d'hydrogène ou un groupe alkyle comportant de 1 à 6 atomes de carbone.

**8.** Composés selon au moins une des revendications de 1 à 5, **caractérisés en ce que** $R^4$ représente un radical $-(CH_2)_m-CH=CH(CH_2)_p- R^6$, dans lequel
m = 0 et p = 1 et
$R^6$ représente un groupe $-OR^5$ ou $-OCOR^5$, dans lequel
$R^5$ représente un atome d'hydrogène ou un groupe alkyle comportant de 1 à 6 atomes de carbone.

**9.** Composés selon au moins une des revendications de 1 à 5, **caractérisés en ce que** $R^4$ représente un radical $-(CH_2)_oC{\equiv}CR^7$,
dans lequel
o = 1 et
$R^7$ représente un groupe alkyle comportant de 1 à 6 atomes de carbone, ou un radical $-OCOR^5$, ou un radical $-CH_2OR^5$, dans lequel $R^5$ représente un radical alkyle comportant de 1 à 6 atomes de carbone ou un atome d'hydrogène.

**10.** Composés selon au moins une des revendications de 1 à 5, **caractérisés en ce que** $R^4$ représente un radical $-(CH_2)_nCH_2Y$,
dans lequel
n = 0 ou 1,
Y représente un atome de F, Cl, Br ou d'iode, un groupe cyano, amino, azido
ou rhodano, ou un radical $-OR^6$, $-SR^6$, $-(CO)SR^6$ ou $-(CO)OR^6$, dans lesquels $R^6$ représente un atome d'hydrogène ou un groupe alkyle comportant de 1 à 10 atomes de carbone.

**11.** Composés selon au moins une des revendications de 1 à 3, **caractérisés en ce que**
$R^3$ et $R^4$ forment ensemble un cycle à cinq ou six chaînons substitué à n'importe quelle position comportant au

moins un atome de carbone et de 0 à 4 hétéroatomes, les hétéroatomes étant issus du groupe de oxygène, soufre et azote.

**12.** Composés selon la revendication 11, **caractérisés en ce qu'**un cycle à cinq chaînons contenant un ou deux hétéroatomes est formé.

**13.** Composés selon la revendication 12, **caractérisés en ce que** le cycle représente un hétérocycle issu du groupe suivant : oxazolidinone, oxazolinone, thiazolidinone, thiazolinone, imidazolidinone, imidazolinone, 1,3-dioxolanone, 1,3-dioxolénone, 1,3-oxathiolanone, 1,3-oxathiolénone, pyrrolidinone, pyrrolinone, oxazolidinethione, oxazolinethione, thiazolidinethione, thiazolinethione, imidazolidinethione, imidazolinethione, dioxolanethione, pyrrolidinethione et pyrrolinethione.

**14.** Composés selon la revendication 13, **caractérisé en ce que** le cycle à cinq chaînons est de l'oxazolidin-3-one ou de l'oxazolidine-2-thione.

**15.** Composés selon la revendication 1, à savoir
4-[17β-méthoxy-17α-(méthoxyméthyl)-3-oxoestra-4,9-diène-11β-yl]-benzaldéhyde-1-(E)-[O-(méthylthio)carbonyl]oxime,
4-[17β-hydroxy-17α-(méthoxyméthyl)-3-oxoestra-4,9-diène-11β-yl]-benzaldéhyde-1-(E)-[O-(méthylthio)carbonyl] oxime,
4-[17β-méthoxy-17α-(méthoxyméthyl)-3-oxoestra-4,9-diène-11β-yl]-benzaldéhyde-1-(E)-[O-(éthylthio)carbonyl] oxime,
4-[17β-méthoxy-17α-(méthoxyméthyl)-3-oxoestra-4,9-diène-11β-yl]-benzaldéhyde-1-(Z)-[O-(éthylthio)carbonylloxime,
4-[17β-méthoxy-17α-(éthoxyméthyl)-3-oxoestra-4,9-diène-11β-yl]-benzaldéhyde-1-(E)-[O-(éthylthio)carbonyl] oxime,
4-[17β-hydroxy-17α-(méthoxyméthyl)-3-oxoestra-4,9-diène-11β-yl]-benzaldéhyde-1-(E)-[O-(n-propylthio)carbonyl]oxime,
4-[17β-méthoxy-17α-(n-propoxyméthyl)-3-oxoestra-4,9-diène-11β-yl]-benzaldéhyde-1-(E)-[O-(méthylthio)carbonyl]oxime,
4-[17β-hydroxy-17α-(i-propoxyméthyl)-3-oxoestra-4,9-diène-11β-yl]-benzaldéhyde-1-(E)-[O-(éthylthio)carbonyl] oxime,
4-[17β-hydroxy-17α-(méthoxyméthyl)-3-oxoestra-4,9-diène-11β-yl]-benzaldéhyde-1-(E)-[O-(éthylthio)carbonyl] oxime,
4-[17β-hydroxy-17α-Z-(3-hydroxypropényl)-3-oxoestra-4,9-diène-11β-yl]-benzaldéhyde-1-(E)-[O-(méthylthio) carbonyl]oxime,
4-[17β-hydroxy-17α-E-(3-hydroxypropényl)-3-oxoestra-4,9-diène-11β-yl]-benzaldéhyde-1-(E)-[O-(méthylthio) carbonyl]oxime,
4-[17β-méthoxy-17α-(3-hydroxy-1-propinyl)-3-oxoestra-4,9-diène-11β-yl]-benzaldéhyde-1-(E)-[O-(éthylthlo)carbonyl]oxime,
4-[17β-hydroxy-17α-(azidométhyl)-3-oxoestra-4,9-diène-11β-yl]-benzaldéhyde-1-(E)-[O-(éthylthio)carbonyl]oxime,
4-[17β-hydroxy-17α-(chlorométhyl)-3-oxoestra-4,9-diène-11β-yl]-benzaldéhyde-1-(E)-[O-(éthylthio)carbonyl]oxime,
4-[17β-éthoxy-17α-(chlorométhyl)-3-oxoestra-4,9-diène-11β-yl]-benzaldéhyde-1-(E)-[O-(méthylthio)carbonyl]oxime,
4-[17β-hydroxy-17α-(cyanométhyl)-3-oxoestra-4,9-diène-11β-yl]-benzaldéhyde-1-(E)-[O-(éthylthio)carbonyl]oxime,
4-[17β-hydroxy-17α-(éthylthiométhyl)-3-oxoestra-4,9-diène-11β-yl]-benzaldéhyde-1-(E)-[O-(éthylthio)carbonyl] oxime,
4-[17β-hydroxy-17α-(éthylthiométhyl)-3-oxoestra-4,9-diène-11β-yl]-benzaldéhyde-1-(E)-[O-(méthylthio)carbonyl] oxime,
4-[17β-éthoxy-17α-(méthylthiométhyl)-3-oxoestra-4,9-diène-11β-yl]-benzaldéhyde-1-(E)-[O-(éthylthio)carbonyl] oxime,
4-[17β-hydroxy-17α-((éthylthiocarbonyl)méthyl)-3-oxoestra-4,9-diène-11β-yl]-benzaldéhyde-1-(E)-[O-(éthylthio) carbonyl]oxime,
4-[17β-hydroxy-17α-(aminométhyl)-3-oxoestra-4,9-diène-11β-yl]-benzaldéhyde-1-(E)-[O-(éthylthio)carbonyl]oxime et

(17R)-4{3-oxoestra-4,9-diène-17-spiro-5'-oxazolidin-2'-one-11β-yl}-benzaldéhyde-1-(E)-[O-(éthylthio)carbonyl] oxime.

**16.** Procédé de fabrication de thiolesters S-substitués de 11β-benzaldoxime-estra-4,9-diène-acide carbonique de formule générale I :

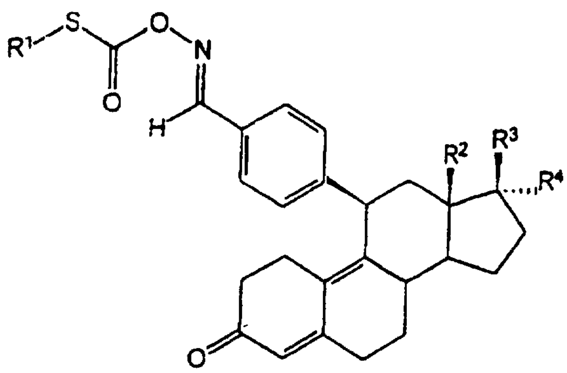

(I)

dans laquelle $R^2$, $R^3$ et $R^4$ ont la signification indiquée dans la revendication 1, **caractérisé en ce qu'**on obtient par transformation de manière connue en soi un composé de formule générale II,

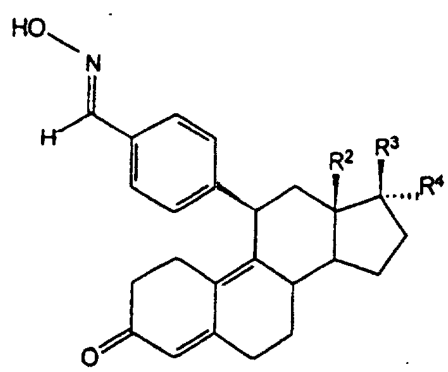

(II)

dans laquelle $R^2$, $R^3$ et $R^4$ ont la même signification que $R^2$, $R^3$ et $R^4$ dans la formule I, par traitement avec un dérivé d'acide formique de formule III

$$\text{Nuc-(CO)-SR}^1 \qquad \text{(III)}$$

dans laquelle $R^1$ a la signification mentionnée dans la revendication 1 et Nuc représente un nucléophile, dans un solvant, et que l'on transforme le composé en un composé de formule générale I.

**17.** Procédé selon la revendication 16, **caractérisé en ce qu'**en tant que solvant, on utilise un amine tertiaire, et qu'on effectue la réaction à une température comprise entre 20°C et 80°C.

**18.** Procédé selon la revendication 17, **caractérisé en ce qu'**on effectue la réaction avec des thioesters d'acide chloroformique dans la pyridine ou le triéthylamine à une température comprise entre 20 et 40°C.

**19.** Compositions pharmaceutiques, **caractérisées en ce qu'**elles contiennent au moins un composé selon une des revendications de 1 à 15.

**20.** Utilisation de composés selon une des revendications de 1 à 15 pour préparer un médicament destiné au traitement de l'endométriose, de l'uterus myomatosus, de la dysménorrhée et du syndrome prémenstruel, à l'induction d'une aménorrhée réversible sans déficit d'oestrogènes et pour la thérapie de substitution climatérique (Hormone Replacement Theraphy, HRT) éventuellement en combination avec des oestrogènes.

**21.** Utilisation des composés selon une des revendications de 1 à 15 pour préparer des contraceptifs.